(19) Europäisches Patentamt
European Patent Office
Office européen des brevets

(11) Publication number: **0 480 714 A2**

# (12) EUROPEAN PATENT APPLICATION

(21) Application number: **91309302.7**

(22) Date of filing: **09.10.91**

(51) Int. Cl.$^5$: **C07C 235/36,** C07C 237/20, A61K 31/16, C07D 335/06, C07D 311/68, C07D 235/14, C07D 295/088, A61K 31/38, A61K 31/35, A61K 31/535

(30) Priority: **11.10.90 US 595913**

(43) Date of publication of application: **15.04.92 Bulletin 92/16**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900 (US)**

(72) Inventor: **Vacca,Joseph P.**
**766 Eisenhauer Drive**
**Telford, PA 18969 (US)**
Inventor: **Veber, Daniel F.**
**290 Batleson Road**
**Ambler, PA 19002 (US)**
Inventor: **Anderson, Paul S.**
**1233 Buttonwood Drive**
**Lansdale, PA 19446 (US)**
Inventor: **Holloway, M. Katharine**
**171 Forest Trail Drive**
**Lansdale, PA 19446 (US)**
Inventor: **Williams, Peter D.**
**260 Shady Nook Road**
**Harleysville, PA 19438 (US)**

(74) Representative: **Barrett-Major, Julie Diane et al**
**Merck & Co., Inc. European Patent Department Terlings Park Eastwick Road**
**Harlow Essex CM20 2QR (GB)**

## (54) HIV protease inhibitors having symmetrical structure.

(57) Compounds of the form,

J-B-B-G-B-B-J

wherein G is a dipeptide isostere, B an amino acid or analog thereof, and J a small terminal group are described. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 480 714 A2

The present invention is concerned with compounds which inhibit the protease encoded by human immunodeficiency virus (HIV). The compounds, or pharmaceutically acceptable salts thereof, are of value in the prevention of infection by HIV, the treatment of infection by HIV and the treatment of the resulting acquired immune deficiency syndrome (AIDS).

The present invention also relates to pharmaceutical compositions containing the compounds and to a method of use of the present compounds and other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Interruption of this processing appears to prevent the production of normally infectious virus. For example, Crawford, S. et al., J. Virol., 53, 899, 1985, demonstrated that genetic deletion mutations of the protease in murine leukemia virus which prevent processing of precursor structural proteins results in non-infectious viral particles. Unprocessed structural proteins also have been observed in clones of non-infectious HIV strains isolated from human patients. These results suggest that inhibition of the HIV protease represents a viable method for the treatment of AIDS and the prevention or treatment of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease.

A particular advantage of the compounds of the present invention is their symmetry. HIV protease is a dimer with a 2-fold axis of symmetry, a property unique to retroviral proteases compared to proteases of mammalian origin. Accordingly, this symmetry should substantially reduce non-specific inhibition causing undesired toxicity in mammals. Also, symmetrical compounds are better able to inhibit HIV protease because they can fit the binding sites better and are also able to bind in either direction along the protease active site domain.

Further, the symmetrical nature of the compounds of the present invention offers the possibility of overcoming the high mutation rate of the HIV virus, for as HIV mutates through its inaccurate reverse transcriptase, the viral protease must still retain the ability to cleave substrates with a 2-fold axis.

## BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Amino Acid |
|---|---|
| Ile | D- or L-isoleucine |
| Val | D- or L-valine |
| | **Activating Agent** |
| HBT (HOBT or HOBt) | 1-hydroxybenzotriazole hydrate |
| DEPC | diethylphosphonyl cyanide |
| HOOBT | 3,4-dihydro-3-hydroxy-4-oxo-1,2,3-benzotriazine |
| | **Condensing Agent** |
| EDC | 1-ethyl-3-(3-dimethylamino-propyl)carbodiimide |
| DCC | dicyclohexylcarbodiimide |
| | **Deprotonating Agents** |
| n-BuLi | n-butyllithium |
| LDA | lithium diisopropylamide |
| LHMDS | lithium hexamethyldisilylazane |
| SHMDS | sodium hexamethyldisilylazane |
| | **Other Reagents** |
| $BF_3 \bullet OEt_2$ | boron trifluoride etherate |
| TEA | triethylamine |

## DETAILED DESCRIPTION OF THE INVENTION AND PREFERRED EMBODIMENTS

This invention is concerned with the use of compounds of formula I, combinations thereof, or pharmaceutically acceptable salts thereof, in the inhibition of HIV protease, the prevention or treatment of infection by HIV and in the treatment of the resulting acquired immune deficiency syndrome (AIDS). Compounds of formula I are defined as follows:

J-B-B-G-B-B-J     I

wherein :

G is

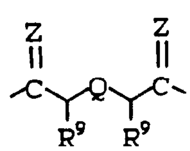

wherein Z is O or S, and

Q is

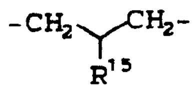 or 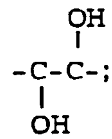;

$R^9$ is independently

1) hydrogen;

2)

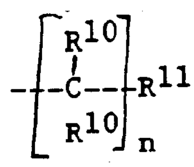 ;

3) $-OR^{11}$,

4) $-N(R^{11})_2$,

5) $C_{1-4}$alkylene-$R^{11}$ or

6) $-S(R^{11})$;

wherein n is 0-5 and $R^{10}$ is independently

a) hydrogen,

b) hydroxy, or

c) $C_{1-4}$-alkyl; $R^{11}$ is

a) hydrogen,

b) : $C_6$-$C_{10}$ aryl, unsubstituted or substituted with one or more of

i) halo,

ii) hydroxy,

iii) $-NH_2$, $-NO_2$, -NHR, or $-NR_2$, wherein R is H, or $C_{1-4}$ alkyl,

iv) $C_{1-4}$ alkyl,

v) $C_{1-3}$ alkoxy,

vi) -COOR,

vii)

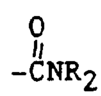,

viii) $-CH_2NR_2$,

ix)

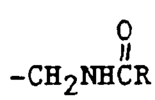,

x) CN,

xi) $CF_3$,

xii)

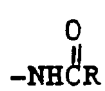,

xiii) aryl $C_{1-3}$ alkoxy,

xiv) aryl,

xv) $-NRSO_2R$,

xvi) $-OP(O)(OR_x)_2$ wherein $R_x$ is H or aryl,
xvii)

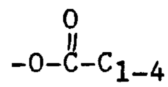

alkyl substituted with one or more of amine or quaternary amine, or
xviii) $-R^{12}$, as defined below;

c) 5 or 6 membered heterocycle including up to 3 heteroatoms selected from N, 0, and S, such as imidazolyl, thiazolyl, furyl, oxazolyl, piperidyl, thiadiazolyl, piperazinyl, pyridyl, or pyrazinyl, any of which heterocycle may be unsubstituted or substituted with one or more of
i) halo,
ii) hydroxy,
iii) $-NH_2$, -NHR, $-NR_2$,
iv) $C_{1-4}$ alkyl,
v) $C_{1-3}$ alkoxy,
vi) -COOR,
vii)

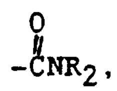

viii) $-CH_2NR_2$,
ix)

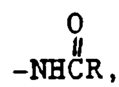

x) -CN,
xi) $CF_3$,
xii) $-NHSO_2R$,
xiii) $-OP(O)(OR_x)_2$ wherein $R_x$ is H or aryl,
xiv)

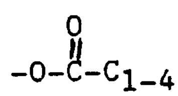

alkyl substituted with one or more of amine or quaternary amine, or
xv) $-R^{12}$;

d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)

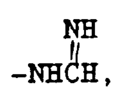

v)

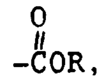

vi) -SR, or arylthio,
xi) $-SO_2NHR$,
vii) $C_{1-4}$ alkyl sulfonyl amino or aryl sulfonyl amino,
viii) -CONHR,
ix)

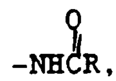

x) -OR,
xi) aryl $C_{1-3}$ alkoxy,
xii) aryl, or
xiii) aryl substituted with $R^{12}$;

e) $C_{3-7}$ cycloalkyl unsubstituted or substituted with one or more of
i) hydroxy,
ii) $C_{1-4}$alkyl,
iii) $-NH_2$, -NHR, $-NHR_2$,
iv)

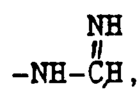

v)

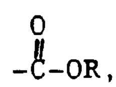

vi) -SR,
vii) $-SO_2NH_2$,
viii) alkyl sulfonylamino or aryl sulfonylamino,
ix) -CONHR,
x)

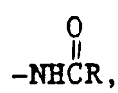

or
xi) $-R^{12}$;

f) a 5- to 7-membered carbocyclic or 7- to 10-membered bicyclic carbocyclic ring which is either saturated or unsaturated, such as cyclopentane, cyclohexane, indan, norbornane, or naphthanene, the carbocyclic ring being unsubstituted or substituted with one or more of
i) halo
ii) -OR, wherein R is H or $C_{1-4}$ alkyl,
iii)

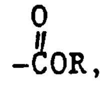

iv)

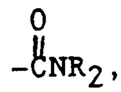

v) $-CH_2NR_2$,
vi) $-SO_2NR_2$ or $-S(O)_y$ R wherein y is 0,1 or 2,
vii) $-NR_2$,
viii)

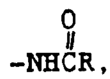

ix) $C_{1-4}$ alkyl,
x) phenyl,
xi) $-CF_3$,
xii)

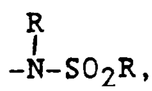

or
xiii) $-R^{12}$; $R^{12}$ is

a) $-X-(CH_2)_m--XR^{13}$ where X is independently -O-,-S-, or NR; m is 2-5, and $R^{13}$ is independently hydrogen or
i) $C_{1-6}$ alkyl,
ii) $C_{1-6}$ alkyl substituted with one or more of
(a) $C_{1-3}$ alkoxy,
(b) -OH,
(c) $-NR_2$ where R is hydrogen or $C_{1-4}$ alkyl;
iii) aromatic heterocycle unsubstituted or substituted with one or more of
(a) $C_{1-4}$ alkyl, or
(b) $-NR_2$;

b) $-X-(CH_2)_m-NR^{13}R^{13}$ wherein $R^{13}$ is the same or different and joined together to form a 5-7 member heterocycle containing up to two additional heteroatoms selected from
(a) -NR,
(b) -O-,
(c) -S-,
(d)

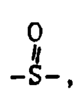

(e) $-SO_2-$;

c) $-(CH_2)_q--NR^{13}R^{13}$ wherein q is 1-5, and $R^{13}$ is defined above;

$R^{15}$ is -OH or $-NHR^{16}$, wherein $R^{16}$ is -H,

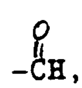

$-C_{1-4}-$ alkyl, or -COOR; and
B is, independently, absent, or

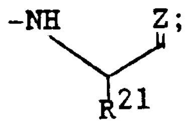

where $R^{21}$ is
a) $-CH(CH_3)_2$,
b) $-CH(CH_3)(CH_2CH_3)$,
c) -Phenyl,
d) $-CH_2CONH_2$, or
e) $-CH_2CH_2OH$;

J is
1) $YR^{17}$ wherein:
Y is O or NH, and
$R^{17}$ is
a) H;
b) $C_{1-6}$ alkyl, unsubstituted or substituted with one or more of
i) $-NR_2$,
ii) -OR,
iii) $-NHSO_2C_{1-4}$ alkyl,
iv) $-NHSO_2$ aryl, or $-NHSO_2$(dialkylaminoaryl),
v) $-CH_2OR$,
vi) $-C_{1-4}$ alkyl,

vii) 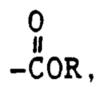

viii) 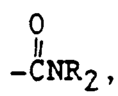

ix) 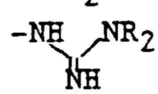 or 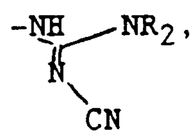

x) 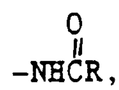

xi) 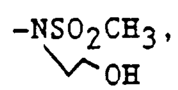

xii) 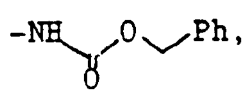

xiii) $-NR_3\oplus$ $A\ominus$ wherein $A\ominus$ is a counterion,
xiv) $-NR^{18}R^{19}$ wherein $R^{18}$ and $R^{19}$ are the same or different and are $C_{1-5}$ alkyl joined together directly to form a 5-7 membered heterocycle containing up to one additional heteroatom selected from -O-, -S-, or -NR-,
xv) aryl,
xvi) -CHO,
xvii) $-OP(O)(OR_x)_2$ wherein $R_x$ is H or aryl, or
xvii)

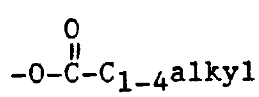

substituted with one or more of amine or quaternary amine, or $-O-[(CH_2)_mO]_n-R$, or $-OP(O)(OR_x)_2$:

2) $N(R^{17})_2$:

3) $-NR^{18}R^{19}$ wherein $R^{18}$ and $R^{19}$ are defined above; or

4)

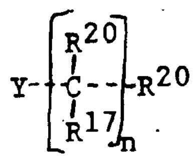

wherein:

Y, $R^{17}$ and n are defined above, and

$R^{20}$ is

a) hydrogen:

b) aryl unsubstituted or substituted with one or more of

i) halo,

ii) -OR, wherein R is H or $C_{1-4}$ alkyl,

iii)

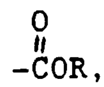

iv)

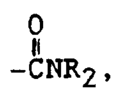

v) $-CH_2NR_2$,

vi) $-SO_2NR_2$,

vii) $-NR_2$,

viii)

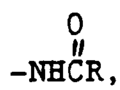

xi) $C_{1-4}$ alkyl,

x) phenyl,

xi) $-CF_3$,

xii)

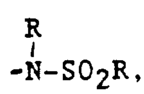

xiii) $-C_{1-4}$ alkyl $-NR_2$,

xiv) $-OP(O)(OR_x)_2$ wherein $R_x$ is H or aryl, or

xv)

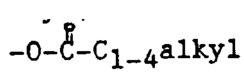

substituted with one or more of amine or quaternary amine or $-OP(O)(OR_x)_2$;

c) A 5-7 membered heterocyclic ring or, 7-10 membered bicyclic heterocyclic ring which is saturated or unsaturated, the ring being unsubstituted or substituted with one or more of

i) halo,
ii) -OR, wherein R is H, $C_{1-4}$alkyl, or $C_{1-4}$ alkenyl,
iii)

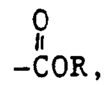

iv)

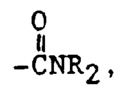

v) $-CH_2NR_2$,
vi) $-SO_2NR_2$,
vii) $-NR_2$,
viii)

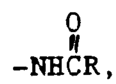

xi) $C_{1-4}$ alkyl,
x) phenyl
xi) $-CF_3$,
xii)

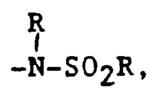

xiii) phenyl $C_{1-4}$ alkyl,
xiv) $-OP(O)(OR_x)_2$ wherein $R_x$ is H or aryl,
xv)

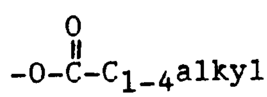

substituted with one or more of amine or quaternary amine, or $-OP(O)(OR_x)_2$, or $-O[(CH_2)_mO]_n-R$, or
xvi)

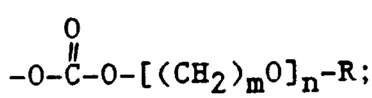

d) A 5 to 7 membered carbocyclic or 7-10 membered bicyclic carbocyclic ring which is either saturated or unsaturated, such as cyclopentane, cyclohexane, indane, norbornane, or naphthalene, the carbocyclic ring being unsubstituted or substituted with one or more of
i) halo,
ii) -OR, wherein R is H or $C_{1-4}$ alkyl,
iii)

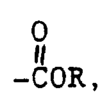

iv) $-CNR_2$,
v)

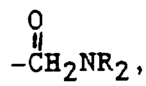

vi) $-SO_2NR_2$,
vii) $-NR_2$,
viii)

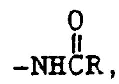

xi) $C_{1-4}$ alkyl,
x) phenyl,
xi) $-CF_3$,
xii)

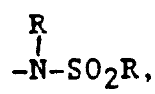

xiii) $-OP(O)(OR_x)_2$ wherein $R_x$ is H or aryl,
xiv)

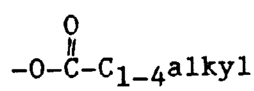

substituted with one or more of amine or quaternary amine, $-OP(O)(OR_x)_2$, or $-O-[(CH_2)_mO]_n-R$, or
xv)

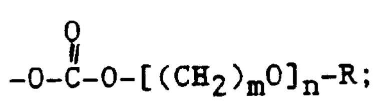

or pharmaceutically acceptable salts thereof.

In a second embodiment of this invention,
G is

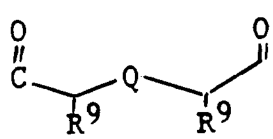

A third embodiment is further limited to compounds, wherein B is absent or present once and Q is

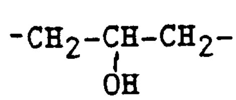 or 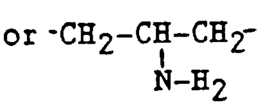 .

A fourth embodiment is further limited to compounds, wherein B is absent and $R^{20}$ is a substituted 5 to 7 membered carbocyclic or heterocyclic or 7 to 10 membered bicyclic carbocyclic or heterocyclic ring which is either saturated or unsaturated. The preferred $R^{20}$ group includes indan substituted with one or more of hydroxy

or amine, benzothiopyranyl substituted one or more times with hydroxy.

Novel compounds of the present invention include, but are not limited to, the following:
N,N′-bis-(2(R)-hydroxy-1(S)-indanyl)-2,6(R,R)-di(phenylmethyl)-4-hydroxy-1,7-heptanediamide,
N,N′-bis-(3(S)-hydroxy-4(S)-benzopyranyl)-2,6(R,R)-di(phenylmethyl)-4-hydroxy-1,7-heptanediamide,
N,N′-bis-(3(S)-hydroxy-4(S)-benzothiopyranyl)-2,6 (R,R)-di(phenylmethyl)-4-hydroxy-1,7-heptanediamide,
N,N′-bis-(4(S)-3,4-dihydro-1H-2-benzothiopyranyl)-2, 6(R,R)-di(phenylmethyl)-4-hydroxy-1,7-heptanediamide,
N,N′-bis-(4(S)-3,4-dihydro-1H-2(R)-oxobenzothiopyranyl)-2,6(R,R)-di(phenylmethyl)-4-hydroxy-1,7-heptanediamide,
N,N′-bis-(4(S)-3,4-dihydro-1H-2,2-dioxobenzothiopyranyl)-2,6(R,R)-di(phenylmethyl)-4-hydroxy-1,7-heptanediamide,
N,N′-bis-(2(S),3(R)-dihydroxy-1(S)-indanyl)-2,6(R,R)-di(phenylmethyl)-4-hydroxy-1,7-heptanediamide,
N,N′-bis-(3(R)-hydroxy-1(S)-indanyl)-2,6(R,R)-di (phenylmethyl)-4-hydroxy-1,7-heptanediamide,
N,N′-bis-(3(R)-amino-2(R)-hydroxy-1(S)-indanyl)-2,6 (R,R)-di(phenylmethyl)-4-hydroxy-1,7-heptanediamide,
N,N′-bis-(4(R)-hydroxy-2(S)-methyl-1(R)-cyclopentyl)-2,6(R,R)-di(phenylmethyl)-4-hydroxy-1,7-heptanediamide,
N,N′-bis-(3(S)-hydroxy-4(S)-benzothiopyranyl-1(S)-oxido)-2,6(R,R)-di(phenylmethyl)-4-hydroxy-1,7-heptanediamide,
N,N′-bis-(2(R)-hydroxy-5(R)-methyl-1(S)-cyclopentyl)-2,6(R,R)-di(phenylmethyl)-4-hydroxy-1,7-heptanediamide,
N,N′-bis-(4(R)-benzothiopyranyl-1(S)-oxido)-2,6(R,R)-di(phenylmethyl)-4-hydroxy-1,7-heptanediamide,
N,N′-bis-(3-methyl-2(S)-amino-1-hydroxybutyl)-2,6-(R,R)-di(phenylmethyl)-4-hydroxy-1,7-heptanediamide,
N,N′-bis-(2(S)-amino-1-hydroxyphenethyl)-2,6(R,R)di(phenylmethyl)-4-hydroxy-1,7-heptanediamide,
N,N′-bis-(2(R)-hydroxy-1(S)-indanyl)-2,6(R,R)-((4-(2(4-morpholinyl)ethoxy)phenyl)methyl)-4-hydroxy-1,7-heptanediamide,
N,N′-bis-(3(S)-hydroxy-4(S)-benzopyranyl)-2,6(R,R)-((4-(2-(4-morpholinyl)ethoxy)phenyl)methyl)-4-hydroxy--1,7-heptanediamide,
N,N′-bis-(3(S)-hydroxy-4(S)-benzothiopyranyl)-2,6-(R,R)-((4-(2-(4-morpholinyl)ethoxy)phenyl)methyl)-4-hydroxy-1,7-heptanediamide,
N,N′-bis-(4(S)-3,4-dihydro-1H-2-benzothiopyranyl)-2,6-(R,R)-((4-(2-(4-morpholinyl)ethoxy)phenyl)methyl)-4-hydroxy-1,7-heptanediamide,
N,N′-bis-(4(S)-3,4-dihydro-1H-2(R)-oxobenzothiopyranyl)-2,6(R,R)-((4-(2-(4-morpholinyl)ethoxy)phenyl) methyl)-4-hydroxy-1,7-heptanediamide,
N,N′-bis-(4(S)-3,4-dihydro-1H-2,2-dioxobenzothiopyranyl)-2,6(R,R)-((4-(2-(4-morpholinyl)ethoxy)phenyl) methyl)-4-hydroxy-1,7-heptanediamide,
N,N′-bis-(2(S),3(R)-dihydroxy-1(S)-indanyl-2,6(R,R)-((4-(2-(4-morpholinyl)ethoxy)phenyl)methyl)-4-hydroxy-1,7-heptanediamide,
N,N′-bis-(3(R)-hydroxy-1(S)-indanyl-2,6(R,R)-((4-(2-(4-morpholinyl)ethoxy)phenyl)methyl)-4-hydroxy-1,7-heptanediamide,
N,N′-bis-(3(R)-amino-2(R)-hydroxy-1(S)-indanyl)-2,6 (R,R)-((4-(2-(4-morpholinyl)ethoxy)phenyl)methyl)-4-hydroxy-1,7-heptanediamide,
N,N′-bis-(4(R)-hydroxy-2(S)-methyl-1(R)-cyclopenyl)-2,6(R,R)-((4-(2-(4-morpholinyl)ethoxy)phenyl)methyl)-4-hydroxy-1,7-heptanediamide,
N,N′-bis-(3(S)-hydroxy-4(S)-benzothiopyranyl-1(S)-oxido)-2,6(R,R)-((4-(2-(4-morpholinyl)ethoxy)phenyl) metyl)-4-hydroxy-1,7-heptanediamide,
N,N′-bis-(2(R)-hydroxy-5(R)-methyl-1(S)-cyclopentyl)-2,6(R,R)-((4-(2-(4-morpholinyl)ethoxy)phenyl)methyl)-4-hydroxy-1,7-heptanediamide,
N,N′-bis-(4(R)-benzothiopyranyl-1(S)-oxido)-2,6(R,R)-((4-(2-(4-morpholinyl)ethoxy)phenyl)methyl)-4-hydroxy-1,7-heptanediamide,
N,N′-bis-(2(R)-hydroxy-1(S)-indanyl)-2,6(R,R)-di((4-((2-hydroxy)ethoxy)phenyl)methyl)-4-hydroxy-1,7-heptanediamide,
N,N′-bis-(3(S)-hydroxy-4(S)-benzopyranyl)-2,6(R,R)-di ((4-((2-hydroxy)ethoxy)phenyl)methyl)-4-hydroxy-1,7-heptanediamide,
N,N′-bis-(3(S)-hydroxy-4(S)-benzothiopyranyl)-2,6-(R,R)-di((4-((2-hydroxy)ethoxy)phenyl)methyl)-4-hydroxy--1,7-heptanediamide,
N,N′-bis-(4(S)-3,4-dihydro-1H-2-benzothiopyranyl)-2,6 (R,R)-di((4-((2-hydroxy)ethoxy)phenyl)methyl)-4-hydroxy-1,7-heptanediamide,
N,N′-bis-(4(S)-3,4-dihydro-1H-2(R)-oxobenzothiopyranyl)-2,6(R,R)-di((4-((2-hydroxy)ethoxy)phenyl)methyl)--4-hydroxy-1,7-heptanediamide,
N,N′-bis-(4(S)-3,4-dihydro-1H-2,2-dioxobenzothiopyranyl)-2,6(R,R)-di((4-((2-hydroxy)ethoxy)phenyl)methyl)-4-hydroxy-1,7-heptanediamide,
N,N′-bis-(2(S),3(R)-dihydroxy-1(S)-indanyl)-2,6(R,R)-di((4-((2-hydroxy)ethoxy)phenyl)methyl)-4-hydroxy-1,7-heptanediamide,
N,N′-bis-(3(R)-hydroxy-1(S)-indanyl)-2,6(R,R)-di((4((2-hydroxy)ethoxy)phenyl)methyl)-4-hydroxy-1,7-heptanediamide,
N,N′-bis-(3(R)-amino-2(R)-hydroxy-1(S)-indanyl)-2,6 (R,R)-di((4-((2-hydroxy)ethoxy)phenyl)methyl)-4-hydroxy-1,7-heptanediamide,
N,N′-bis-(4(R)-hydroxy-2(S)-methyl-1(R)-cyclopentyl)2,6(R,R)-di((4-((2-hydroxy)ethoxy)phenyl)methyl)-4-hydroxy-1,7-heptanediamide,
N,N′-bis-(3(S)-hydroxy-4(S)-benzothiopyranyl-1(S)-oxido)-2,6(R,R)-di((4-((2-hydroxy)ethoxy)phenyl) methyl)-4-hydroxy-1,7-heptanediamide,
N,N′-bis-(2(R)-hydroxy-5(R)-methyl-1(S)-cyclopentyl)-2,6(R,R)-di((4-((2-hydroxy)ethoxy)phenyl)methyl)-4-hydroxy-1,7-heptanediamide,
N,N′-bis-(4(R)-benzothiopyranyl-1(S)-oxido-2,6(R,R)-di((4-((2-hydroxy)ethoxy)phenyl)methyl)-4-hydroxy-1,7-heptanediamide,
N,N′-bis-(3-methyl-2(S)-amino-1-hydroxybutyl)-2,6(R,R)-di((4-((2-hydroxy)ethoxy)phenyl)methyl)-4-hydroxy, 7-heptanediamide, or
N,N′-bis-(2(S)-amino-1-hydroxyphenethyl)-2,6(R,R)-di((4-((2-hydroxy)ethoxy)phenyl)methyl)-4-hydroxy-1,7-heptanediamide, or
pharmaceutically acceptable salts thereof.

Preferred embodiments of the present invention include the following compounds.

A.

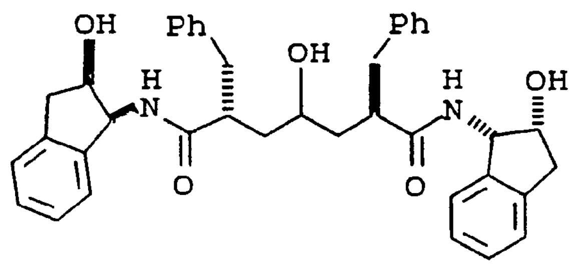

N,N′-bis-(2(R)-hydroxy-1(S)-indanyl)-2,6(R,R)-di(phenylmethyl)-4-hydroxy-1,7-heptanediamide;

B.

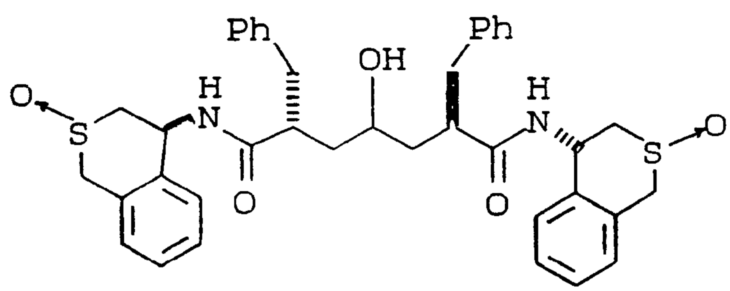

N,N′-bis-(4(S)-3,4-dihydro-1H-2(R)-oxobenzothiopyranyl)-2,6(R,R)-di(phenylmethyl)-4-hydroxy-1,7-heptanediamide;

C.

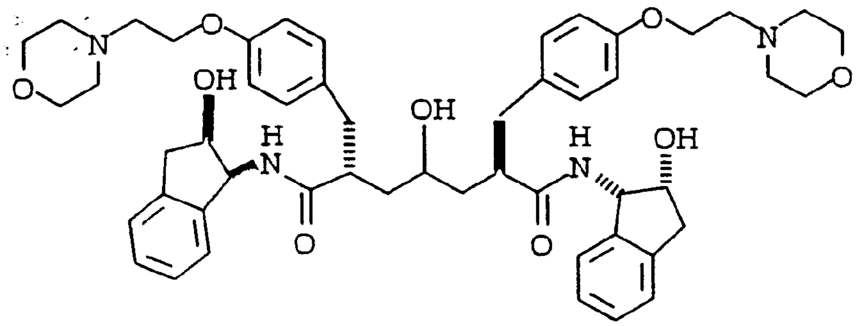

N,N′-bis-(2(R)-hydroxy-1(S)-Indanyl)-2,6(R,R)-di((4-2(4-morpholinyl)ethoxy)phenyl)methyl)-4-hydroxy-1,7-heptanediamide; or

D.

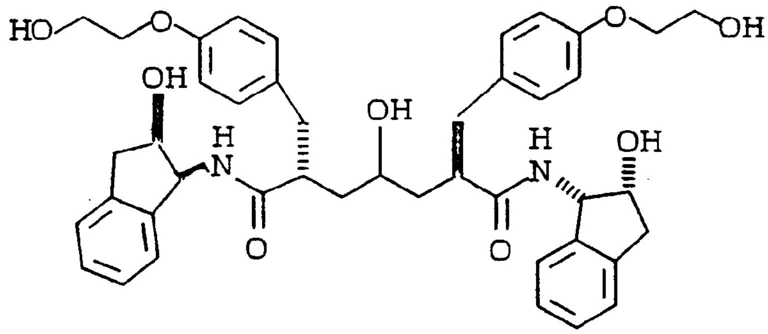

N,N′-bis-(2(R)-hydroxy-1(S)-indanyl)-2,6(R,R)-di((4-((2-hydroxy)ethoxy)phenyl)methyl)-4-hydoxy-1,7-heptanediamide,

or pharmaceutically acceptable salts thereof.

In the compounds of the present invention, the G, B and J components and the like may have asymmetric centers and occur as racemates, racemic mixtures and as individual diastereomers, with all isomeric forms being included in the present invention.

When any variable (e.g., aryl, heterocycle, R, $R^9$, $R^{10}$, $R^{11}$,$R^{12}$, $R^{15}$, $R^{16}$, $R^{17}$, n, Z, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge. "Alkenyl" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle or heterocyclic, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements

include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The pharmaceutically-acceptable salts of the compounds of Formula I (in the form of water- or oil-soluble or dispersible products) include the conventional non-toxic salts or the quaternary ammonium salts of these compounds, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with Schemes I-V. In general, once the G substituent is made, the rest of the synthesis follows the principle of amide bond formation by the coupling methods of either solution-phase or solid-phase peptide synthesis. The addition and removal of one or more protecting groups is also typical practice.

Amide couplings used to form the compounds of this invention are typically performed by the carbodiimide method with reagents such as dicyclohexylcarbodiimide, or 1-ethyl-3-(3-dimethylaminopropyl) carbodiimide. Other methods of forming the amide or peptide bond include, but are not limited to synthetic routes via an acid chloride, azide, mixed anhydride or activated ester. Typically, solution phase amide couplings are performed, but solid-phase synthesis by classical Merrifield techniques may be employed instead.

Some schemes for preparing compounds of formula I are presented below. Examples 1-10 specifically illustrate the application of the following schemes to specific compounds. Additional related information on synthetic background is contained in EP 0337714, herein inccoporated by reference for these purposes.

SCHEME I

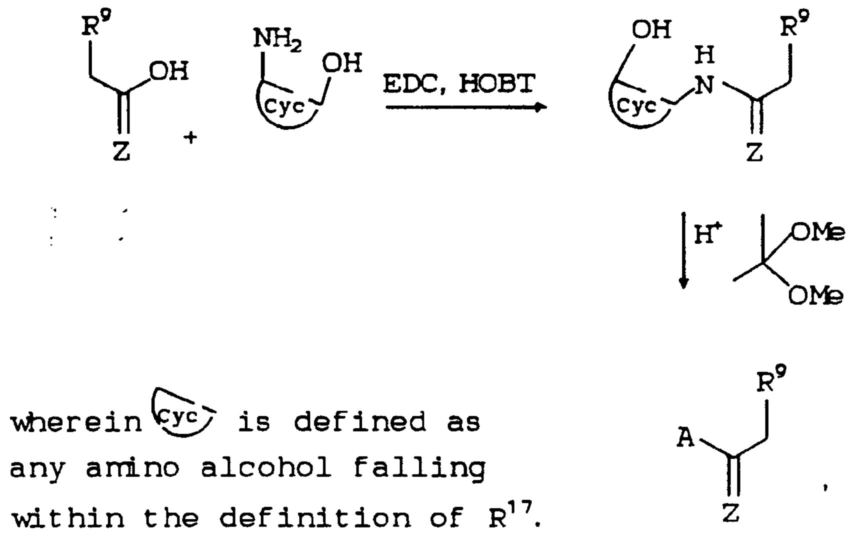


A principal reactant

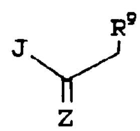
1

is prepared by typical carbodiimide coupling reactions. In Scheme I, for instance, 1-ethyl-3-(3-dimethylaminopropyl)carbodiimide and the activator HOBT are employed to yield a subspecies of 1,

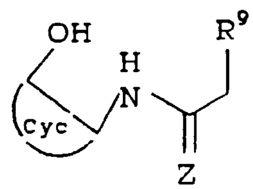
2.

DCC and other activators, such as HOOBT (3,4-dihydro-3-hydroxy-4-oxo-1,2,3-benzotriazine) or DEPC are feasible alternate reagents. Another method of coupling is via acid chlorides with, e.g. methylene chloride and triethylamine.

If a protecting group for substituent J is needed, as is the case for 2, it can be added at this point prior to subsequent alkylation steps. Thus 2 is protected by cyclization via catalysis with a strong acid in the presence of, e.g., dimethoxypropane, to yield

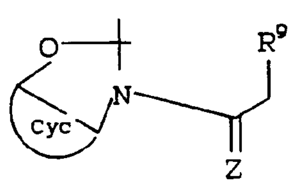
3,

or more generally

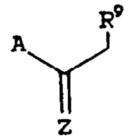

4.

Strong acids such as p-toluene sulfonic acid or camphorsulfonic acid are suitable acid catalysts.

The reactant 1 is a suitable substitute for 4 in the reaction schemes that follow, but only in those cases when J is not in need of a protecting group.

Illustrations of 4, the product of Scheme I are found in Table 1.

TABLE I

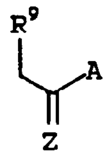

4

| $R^9$ | Z | A |
|---|---|---|
| $CH_2Ph$ | O | 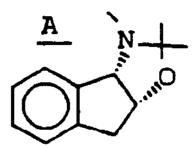 |
| $CH_2Ph$ | O | 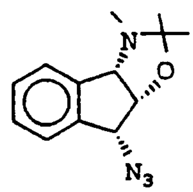 |
| $CH_2Ph$ | O | 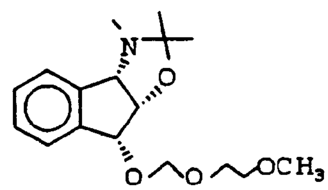 |
| $CH_2Ph$ | O | 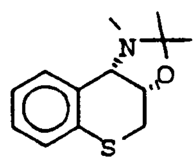 |
| $CH_2Ph$ | O | 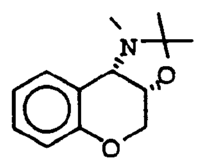 |

TABLE I CONT'D

| $R^9$ | Z | A |
|---|---|---|
| $CH_2Ph$ | O | 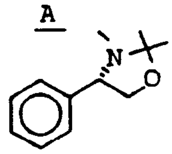 |
| 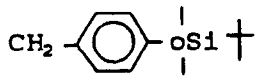 | O | 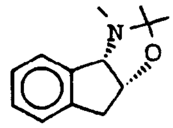 |
| 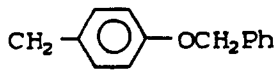 | O | 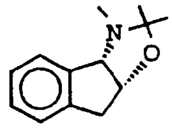 |
| 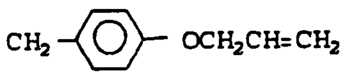 | O | 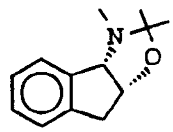 |
| $CH_3CH_2$ | O | 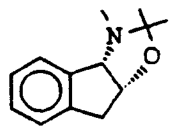 |
| O-Ph | O | 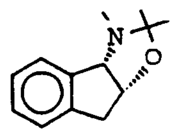 |
| 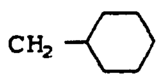 | O | 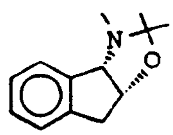 |

TABLE I CONT D

| $R^9$ | Z | A |
|---|---|---|
| $CH_2-CH=CH-Ph$ | O | 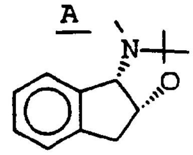 |
| $CH_2-CH=CH-$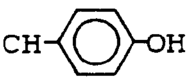 | O | — " — |
| $CH_2-CH=CH-$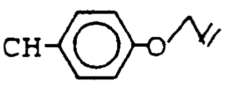 | O | — " — |
| $CH_2-CH=CH-$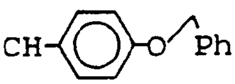 | O | — " — |
| $CH_2-CH=CH-$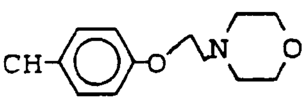 | O | — " — |
| S-Ph | O | — " — |

TABLE I CONT D

| $R^9$ | Z | A |
|---|---|---|
| $CH_2Ph$ | O | 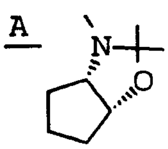 |
| $CH_2Ph$ | O | 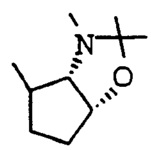 |
| $CH_2Ph$ | O | 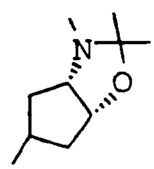 |
| $CH_2Ph$ | O | 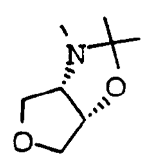 |
| $CH_2-CH=CH_2$ | O | 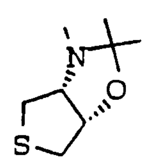 |

A first method for forming products of general formula I is shown in Scheme II.

SCHEME II

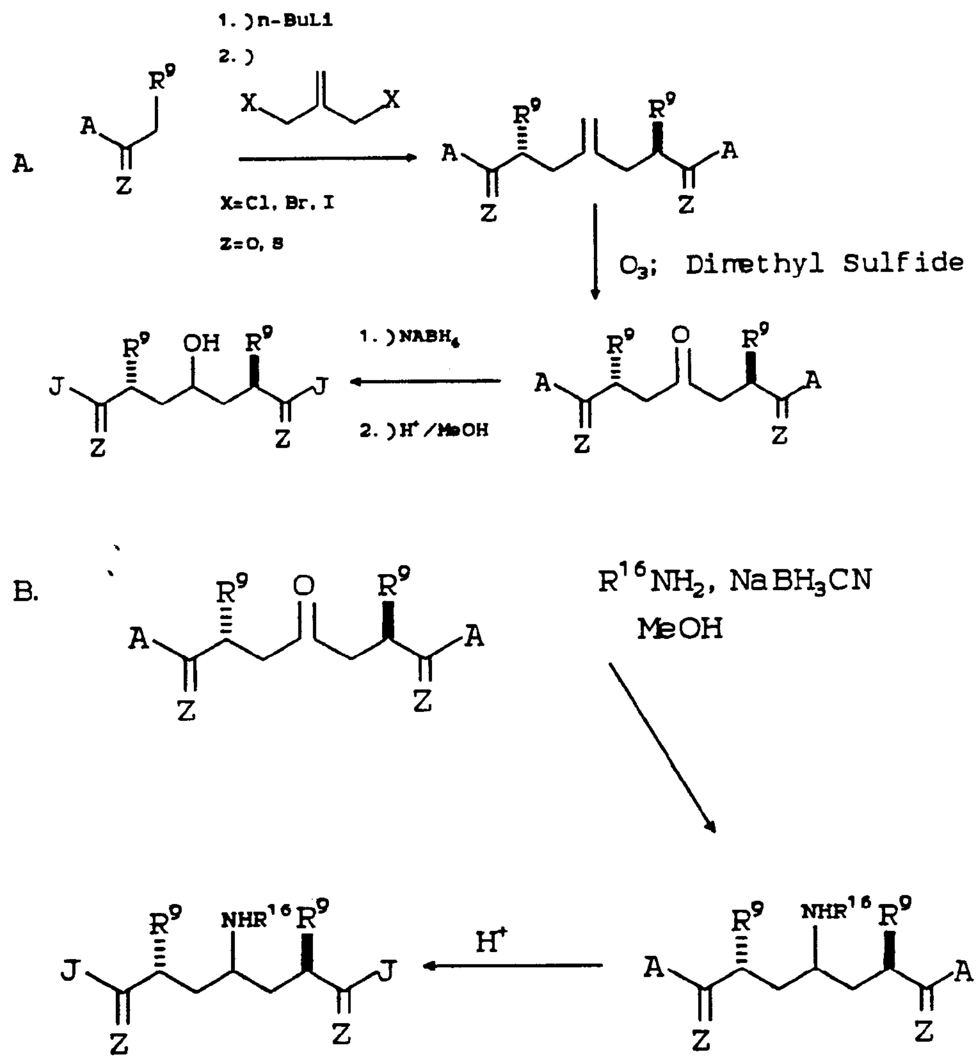


In Scheme IIA, alkylation of 4 is performed by a first step of deprotonation of 4 with n-butyllithium or lithium diisopropylamide (LDA), followed by a second step of adding an alkenyl halide, to a afford

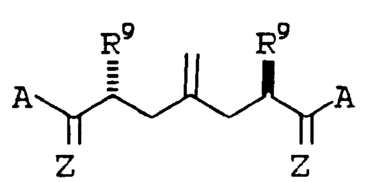

5.

Ozonolysis of 5, then cleavage of the resulting ozonide with dimethylsulfide (DMS) produces a central carbonyl,

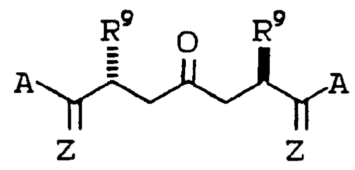

6.

Reduction with, for example, $NaBH_4$, followed by removal of the protecting group(s) produces compounds of the present invention wherein Q is $-CH_2CHOHCH_2-$. Removal of ketals in the A substituent can be accomplished by treatment with acid in the presence of methanol, or by aqueous acid or by 1N HCl in THF.

In Scheme IIB, the synthesis of formula I compounds having Q is $-CH_2CH(NHR^{16})CH_2-$ is conveniently accomplished by reductive amination of 6, followed by deprotection procedures already discussed.

A second method for forming compounds of general formula I is shown in Scheme III.

## SCHEME III

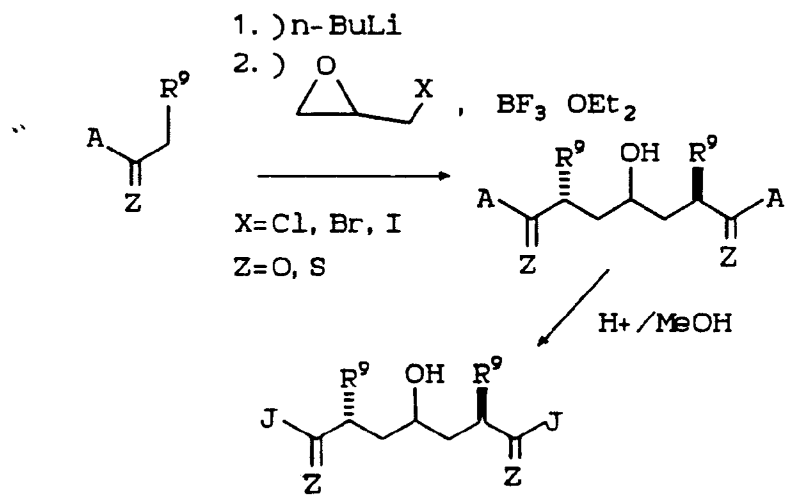


A second method of producing formula I compounds is provided by Scheme III. Reactant 4 is subjected to Lewis acid catalyzed alkylation by deprotonation with n-butyllithium, followed by alkylation with an epihalohidrin in the presence of boron trifluoride etherate. Deprotection follows, as needed.

A third method for forming compounds of general formula I is shown in Scheme IV.

SCHEME IV

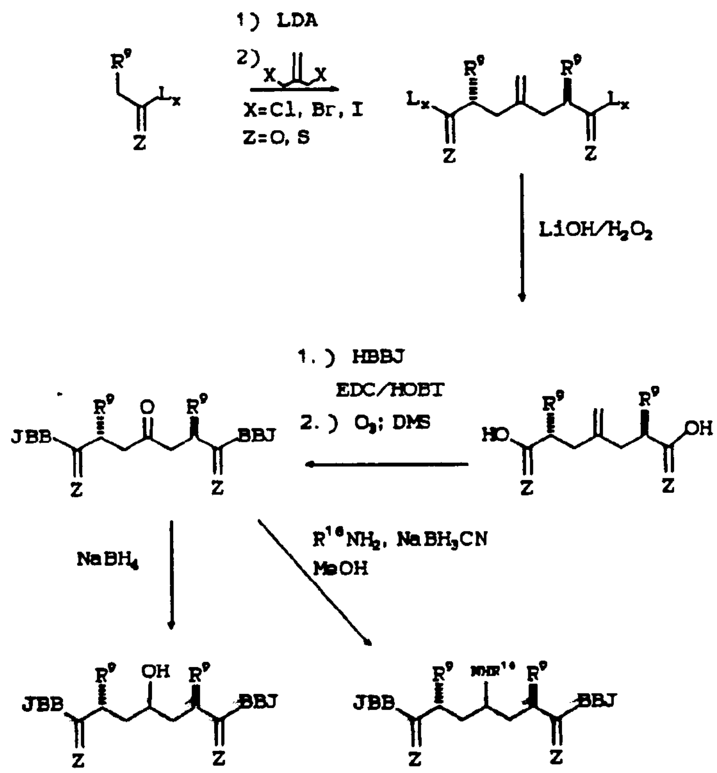


($L_x$ is a typical chiral auxiliary used to perform chiral bond formations. For a recent use, see Evans, D.A. et al., J. Am. Chem. Soc. 112, 5290-5313 (1990).)

A third method of synthesizing formula I compounds according to Scheme IV employs a chiral auxiliary $L_x$ instead of protected J (or A), for the purpose of increasing yields. The reactant

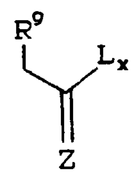

7

is alkylated by a procedure similar to the first step of Scheme II, above. Mild deprotonation agents such as LDA, LHMDS or SHMDS are reacted with 7, followed by alkylation with added alkylene halide, to yield

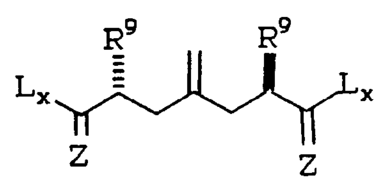

8.

One illustration of a chiral auxiliary $L_x$ is the oxazolidinone substituent of Example 5.

Removal of the auxiliary with base hydrolysis is accomplished preferably by LiOH and $H_2O_2$, less preferably with LiOH or NaOH. Subsequent coupling by the carbodiimide method, ozonolysis, reduction or reductive amination provides compounds of formula I as outlined in Scheme IV.

Illustrations of the product of Schemes I-IV include the following compounds of Table II.

## Table II

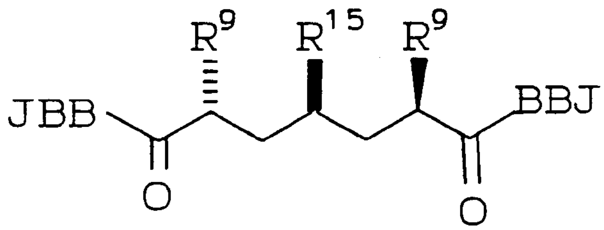

| $R^9$ | $R^{15}$ | BBJ |
|---|---|---|
| $CH_2$- Ph | OH | 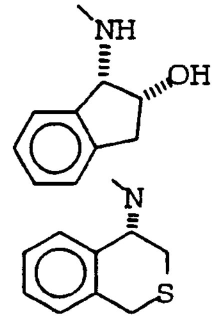 |
| $CH_2$- Ph | OH | |
| $CH_2$-Ph | OH | 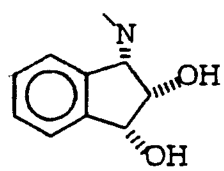 |
| $CH_2$-Ph | OH | 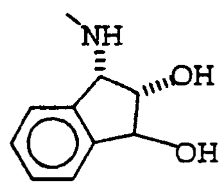 |

Table II cont'd

| $R^9$ | $R^{15}$ | BBJ |
|---|---|---|
| $CH_2$- Ph | OH | 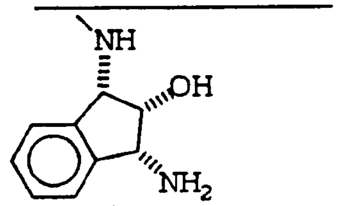 |
| $CH_2$- Ph | $NH_2$ | 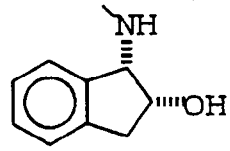 |
| $CH_2$-Ph | OH | 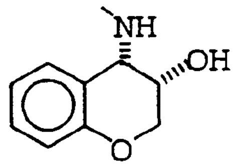 |
| $CH_2$-Ph | OH | 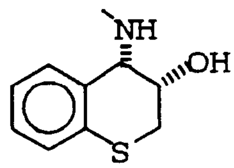 |

Table II cont'd

| R⁹ | $R^{15}$ | BBJ |
|---|---|---|
| $CH_2-Ph$ | $NH_2$ | 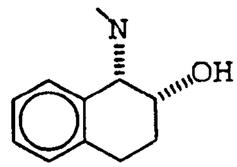 |
| $CH_2-Ph$ | OH | 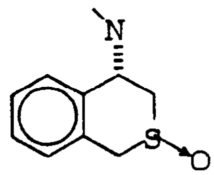 |
| $CH_2-Ph$ | OH | 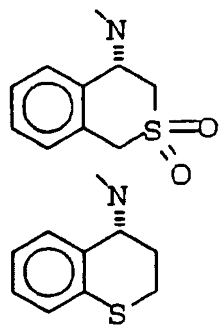 |
| $CH_2-Ph$ | OH | |
| 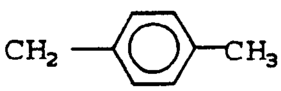 | OH | 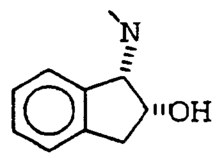 |
| 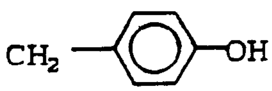 | OH | 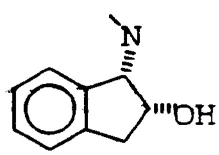 |
| 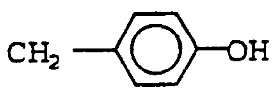 | OH | 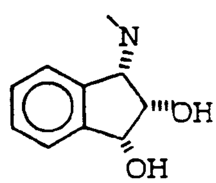 |

## Table II cont'd

| $R^9$ | $R^{15}$ | BBJ |
|---|---|---|
| $CH_2$ 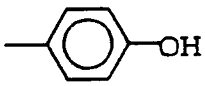 | $NH_2$ | 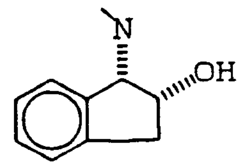 |
| $CH_2$ 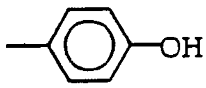 | $NH_2$ | 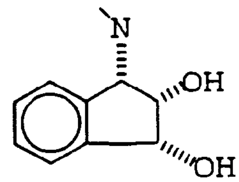 |
| $CH_2$ 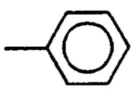 | OH | -Ile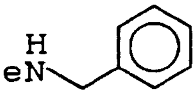 |
| $CH_2$ 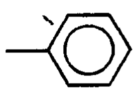 | OH | -Ile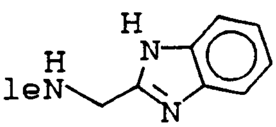 |
| $CH_2$ 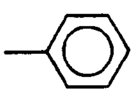 | $NH_2$ | -Ile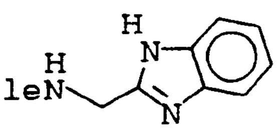 |
| $CH_2$ 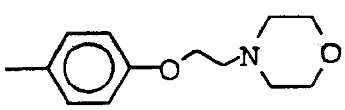 | OH | -Ile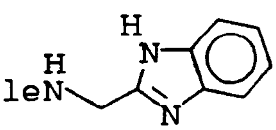 |
| $CH_2$ 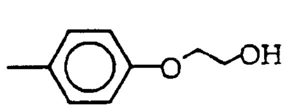 | OH | -Ile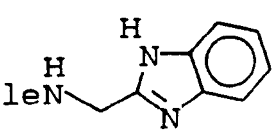 |

Table II cont'd

| $\underline{R^9}$ | $\underline{R^{15}}$ | BBJ |
|---|---|---|
| 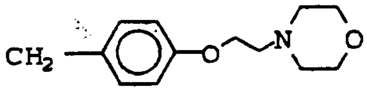 | $NH_2$ | 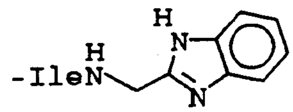 |
| 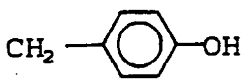 | OH | 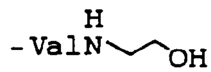 |
| 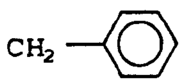 | OH | 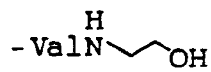 |
| 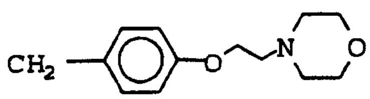 | OH | 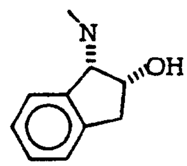 |
| 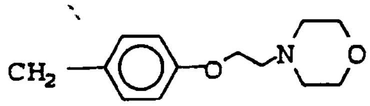 | $NH_2$ | 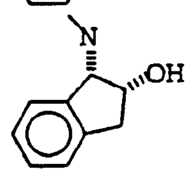 |
| 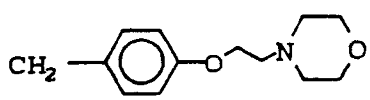 | OH | 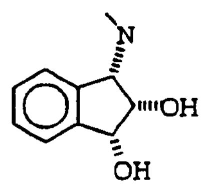 |

Table II cont'd

| $R^9$ | $R^{15}$ | BBJ |
|---|---|---|
| 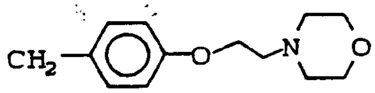 | OH | 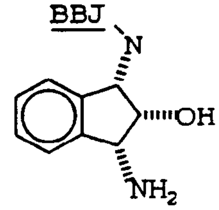 |
| 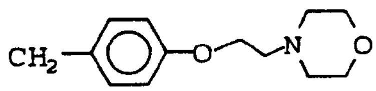 | $NH_2$ | 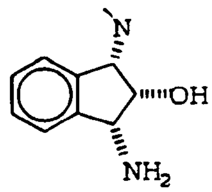 |
| 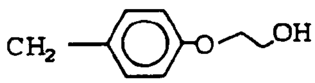 | OH | 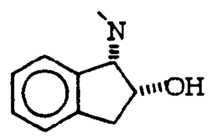 |
| 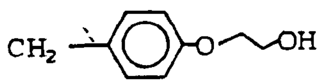 | OH | 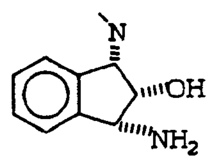 |
| 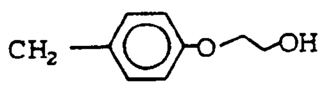 | $NH_2$ | 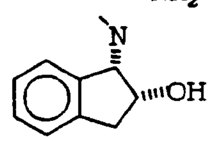 |
| 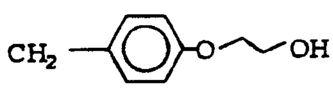 | $NH_2$ | 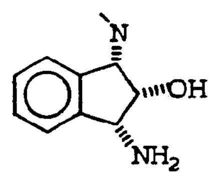 |
| 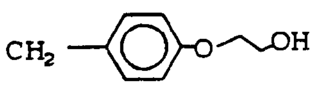 | OH | 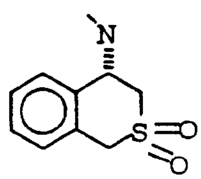 |

## Table II cont'd

| $R^9$ | $R^{15}$ | BBJ |
|---|---|---|
| $CH_2CH=CH=Ph$ | OH | 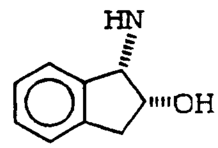 |
| $CH_2CH=CH-Ph$ | OH | 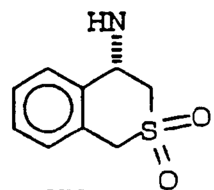 |
| $CH_2CH=CH-$ 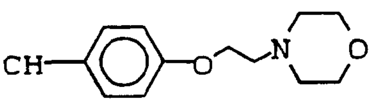 | OH | 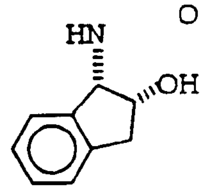 |
| $CH_2CH=CH-$ 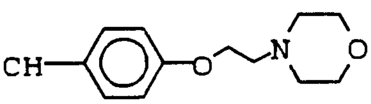 | OH | 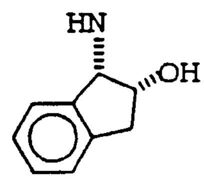 |

A general scheme for synthesizing different $R^9$ groups is shown below.

## SCHEME V

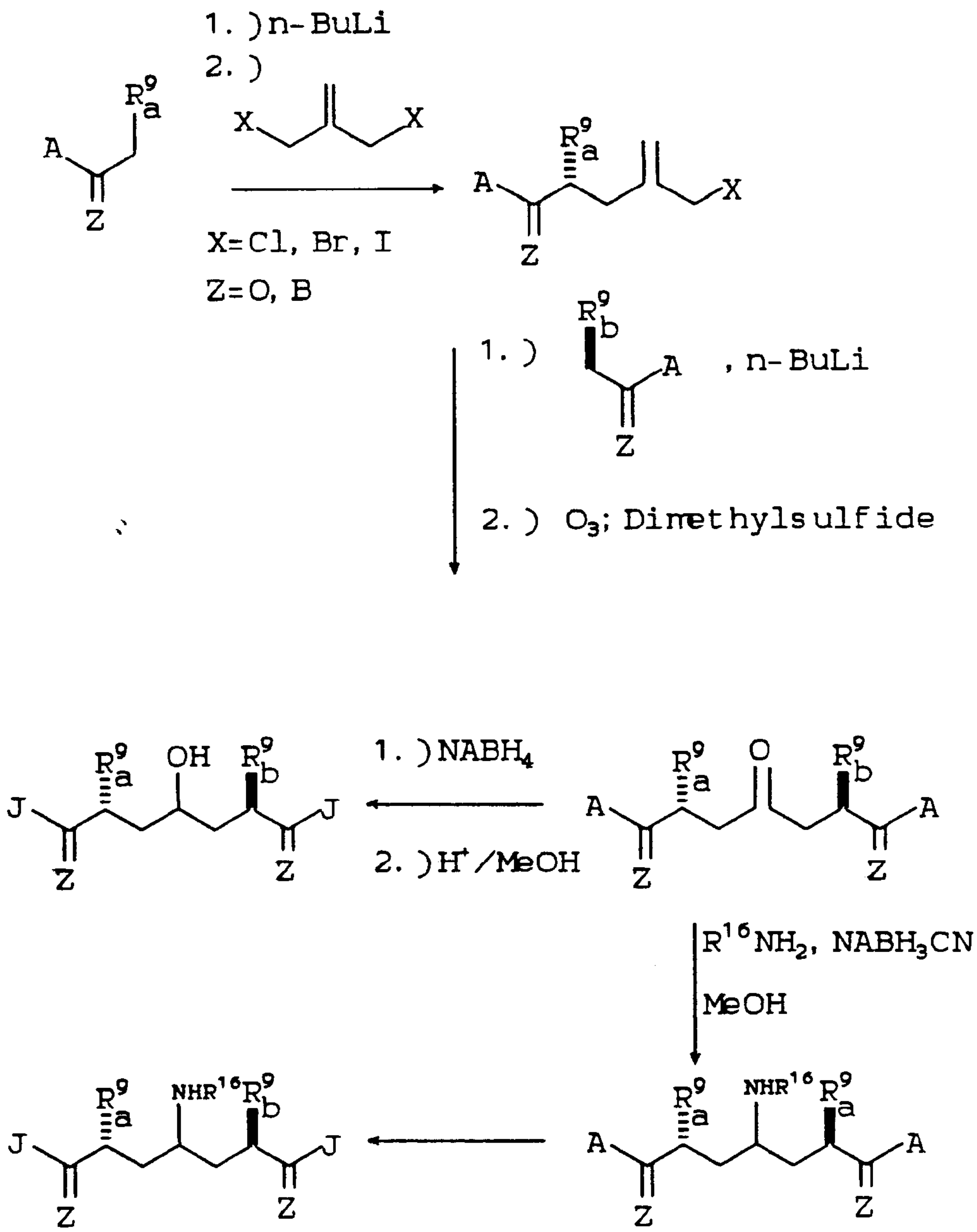


Scheme V provides a method for synthesizing formula I compounds having different $R^9$ groups. It is substantially the same as Scheme II, except that initial alkylation of 4 is divided into two separate steps corresponding to $R^9_a$ and to $R^9_b$.

Illustrations of compounds made by the methods of Schemes I-V are found in Table III.

## Table III

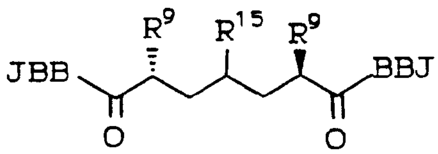

| $R^9$ | $R^{15}$ | BBJ or BJ or J |
|---|---|---|
| 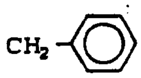 | OH | 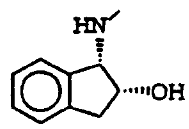 |
| 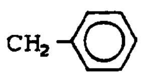 | OH | 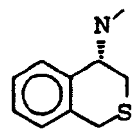 |
| $CH_2$-Ph | OH | 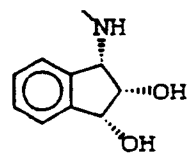 |
| $CH_2$-Ph | OH | 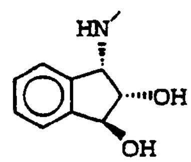 |
| $CH_2$-Ph | OH | 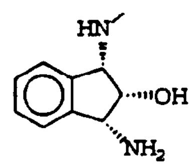 |
| $CH_2$-Ph | $NH_2$ | 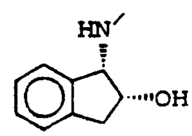 |

| $R^9$ | $R^{15}$ | BBJ or BJ or J |
|---|---|---|
| $CH_2$-Ph | OH | 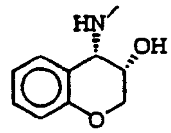 |
| $CH_2$-Ph | OH | 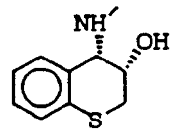 |
| $CH_2$-Ph | $NH_2$ | 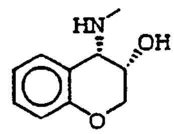 |
| $CH_2$-Ph | OH | 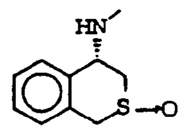 |
| $CH_2$-Ph | OH | 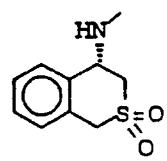 |
| $CH_2$-Ph | OH | 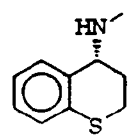 |
| $CH_2$- Ph-$CH_3$ | OH | 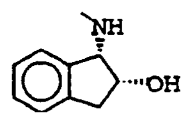 |

| $R^9$ | $R^{15}$ | BBJ or BJ or J |
|---|---|---|
| 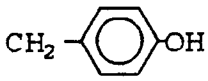 | OH | 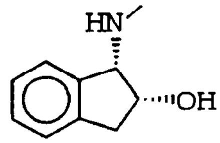 |
| 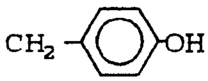 | OH | 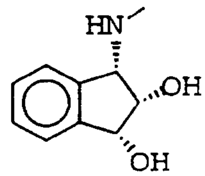 |
| 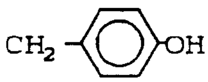 | $NH_2$ | 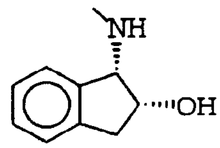 |
| 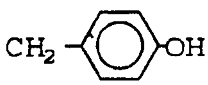 | $NH_2$ | 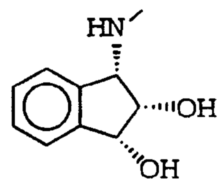 |
| 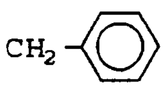 | OH | -Ile 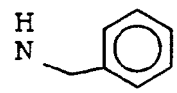 |
| 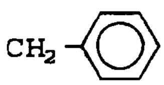 | OH | -Ile 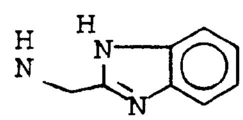 |

| $R^9$ | $R^{15}$ | BBJ or BJ or J |
| --- | --- | --- |
| 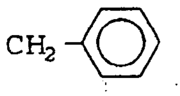 | $NH_2$ | -Ile 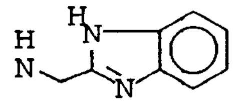 |
| 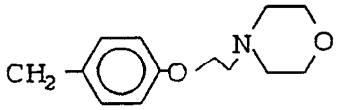 | OH | " |
| 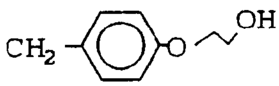 | OH | " |
| 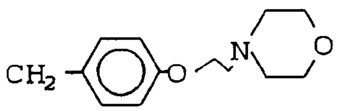 | $NH_2$ | " |
| 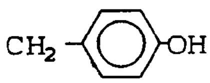 | OH | -Val 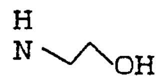 |
| 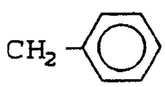 | OH | -Val 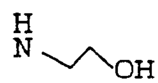 |

| $R^9$ | $R^{15}$ | BBJ or BJ or J |
|---|---|---|
| 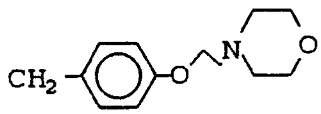 | OH | 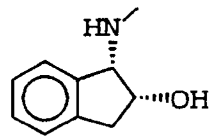 |
| ——" —— | $NH_2$ | 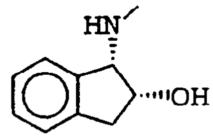 |
| ——" —— | OH | 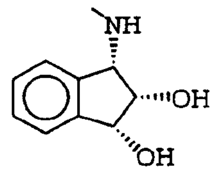 |
| ——" —— | OH | 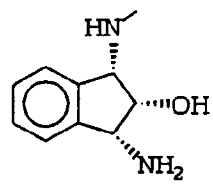 |
| ——" —— | $NH_2$ | ——" —— |
| 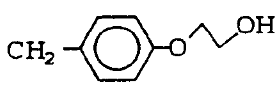 | OH | 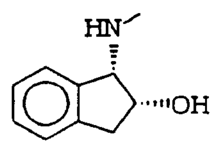 |
| 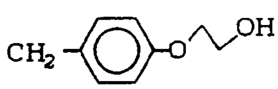 | OH | 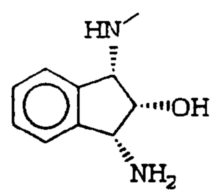 |

| $R^9$ | $R^{15}$ | BBJ or BJ or J |
|---|---|---|
| — " — | $NH_2$ | 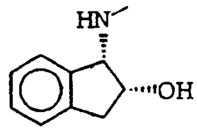 |
| — " — | $NH_2$ | 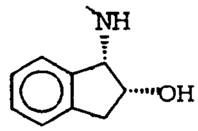 |
| — " — | OH | 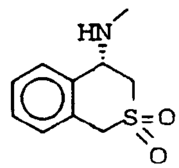 |
| $CH_2CH=CH-Ph$ | OH | 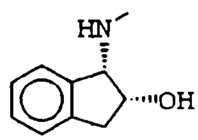 |
| $CH_2CH=CH-Ph$ | OH | 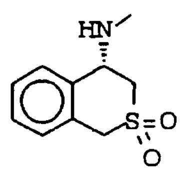 |
| 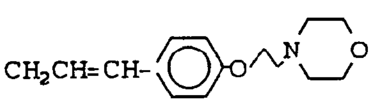 | OH | 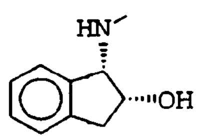 |
| 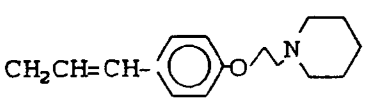 | OH | — " — |

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymtomatic, and actual or potential exposure to IV. For example, the compounds of this invention are useful in treating infection by HIV after suspected past exposure to HIV by e.g., blood transfusion, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age of the patient, body weight, general health, sex, diet, mode and time of administration, rate of excretion, drug combination, the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the following Table [source: Marketletter, Nov. 30. 1987, pp. 26-27; Genetic Engineering News, Jan. 1988, Vol. 8, 23];

TABLE[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| ddI (dideoxyinosine) | Bristol-Myers | AIDS, ARC |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
| --- | --- | --- |
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological involvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP KS (bropirimine) | Upjohn | Advanced AIDS, |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |

| Drug Name | Manufacturer | Indication |
|---|---|---|
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

EXAMPLE 1

Preparation of N,N-bis-(2(R)-hydroxy-1(S)-indanyl)-2, 6(R,R)diphenylmethyl-4-hydroxy-1,7-heptanediamide

Step 1: Preparation of N-(2(R)-hydroxy-1(S)-indanyl)-3-phenyl-propaneamide

To a cold (0°C) solution of methylene chloride (30 ml) containing 2(R)-hydroxyl-1(S)-aminoindane (750 mg, 5.0 mmol) and triethylamine (606 mg, 6.0 mmol) was added a solution of hydrocinnamoyl chloride (843 mg,

5.0 mmol) in 5 ml of methylene chloride. After 2 hours the reaction was poured into a separatory funnel containing 50 ml of methylene chloride and washed with 10% citric acid solution (2 x 30 ml). The organic layer was dried, filtered and concentrated to afford a white solid.

Step 2: Preparation of N-(2(R)-hydroxy-1(S)-indan(N,O-isopropylidene)-yl)-3-phenyl-propaneamide

The crude white solid from Step 1 above was dissolved in 50 ml of methylene chloride and 5 ml of dimethoxypropane was added followed by the addition of 100 mg of p-toluenesulfonic acid. The reaction was stirred at room temperature for 18 hours and then poured into a separatory funnel and washed with saturated $NaHCO_3$ solution (2 x 30 ml). The organic layer was dried, filtered and concentrated to afford an oil which was chromatographed ($SiO_2$, 40% EtOAc/Hexane) to give an oil which eventually crystallized.

Step 3: Preparation of N-(2(R)-hydroxy-1(S)-indan(N,O-isopropylidene)-yl)-2(R)-phenylmethyl-4-((3-(N′-(2(R)-hydroxy-1(S)-indan-(N,O-isopropylidene)-yl)-2(R)-phenylmethyl-propaneamide)-pent-5-eneamide

To a solution of N-(2(R)-hydroxy-1(S)-indan(N,O-isopropylidene)-yl)-3-phenyl-propaneamide (353 mg, 1.0 mmol) in 5 ml of THF cooled to -78°C was added 0.75 ml (1.2 mmol) of a 1.6 N n-BuLi solution in hexanes. After 30 minutes, 60 mg (0.48 mmol) of 3-chloro-2-chloromethyl-1-propene was added, the solution was stirred at -78°C for 1 hour, warmed to 0°C, stirred another hour and then quenched by pouring into 30 ml of 10% citric acid solution. This was washed with ethyl acetate (2 x 40 ml) and the organic layer washed with satd. brine soln., dried filtered and concentrated. The crude residue was chromatographed on a prep plate (2 mm $SiO_2$, 40% EtOAc/Hexane) to afford 120 mg of product.

Step 4: Preparation of N,N-bis-(2(R)-hydroxy-1(S)-indan-N,O-isopropylidene-2,6(R,R)-diphenylmethyl-4-keto-1,7-heptanediamide

The product from Step 3 above (119 mg, 171 mmol) was dissolved in 6 ml of methylene chloride and 2 ml of methanol, cooled to -78°C, and ozone bubbled through until it was saturated as indicated by the color of blue. The reaction was purged with $N_2$ to remove the ozone and 0.040 ml of dimethylsulfide was added. The reaction was warmed to room temperature and stirred for 18 hours after which time it was concentrated to dryness to give the crude product.

Step 5: Preparation of N,N-bis-(2(R)-hydroxy-1(S)indan-(N,O-isopropylidene)yl)-2,6(R,R)-diphenyl)methyl-4-hydroxy-1,7-heptanediamide

The product from Step 4 above was dissolved in 10 ml of methanol and 120 mg of $NaBH_4$ added. After 2 hours at room temperature, the reaction was diluted with 30 ml of water, the methanol was concentrated off on the roto-evaporator and the aqueous layer extracted with ethyl acetate (2 x 30 ml) which was dried filtered and concentrated to afford a crude product. This was chromatographed (2 mm prep plate, $SiO_2$, 50% Et0Ac/Hexane) to afford 70 mg of product as a foam.

Step 6: Preparation of N,N-bis-(2(R)-hydroxy-1(S)-indanyl)2,6(R,R)-diphenylmethyl-4-hydroxyl,7-heptanediamide

The product from Step 4 above, N,N-bis-(2(R)-hydroxy-1(S)-indan-(N,O-isopropylidene)yl)-2,6(R,R)-diphenylmethyl-4-hydroxy-1,7-heptanediamide, was dissolved in 10 ml of methanol and 14 mg of p-toluenesulfonic acid was added. The reaction was stirred at room temperature for 18 hours after which a white precipitate had formed. This was filtered, washed with methanol and dried to afford 30 mg of the desired product.

EXAMPLE 2

Preparation of N,N-bis(2(R)-hydroxy-1(S)-indanyl)2,6(R,R)-di((4′-hydroxyphenyl)methyl)-4-hydroxy-1,7-heptanediamide

Step 1: Preparation of N-(2(R)-hydroxy-1(S)-indanyl)-3(4-hydroxyphenyl)propaneamide

A quantity of 3-(4-hydroxyphenyl)propionic acid (3.68 g, 22.15 mm) was dissolved in 50 ml of DMF, and to it was added HOBT (2.99 g, 22.15 mm), EDC (4.25 g, 22.15 mm), and 2(R)-hydroxy-1(S)aminoindane (3 g,

20.13 mm) respectively. Triethylamine (3.09 ml, 22.15 mm) was added to the stirring solution to a pH of 8.5. After stirring for 18 hours at room temperature, the DMF was removed and the residue was partitioned between 350 ml EtOAc/100 ml $H_2O$. The organic layer was washed 1 x 100 ml sat. $NaHCO_3$, 1 x 100 ml brine, dried with $Na_2SO_4$ and concentrated to give 5.9 g of crude product. The crude product was triturated with $E_2O$=1$CH_2Cl_2$ (20 ml, 20 ml) to give 4.95 g of pure product.

Step 2: N-(2(R)-hydroxy-1(S)-indan-(N,O-isopropylidene)yl)-3(4-hydroxyphenyl)propaneamide

The product from Step 1 (4.95 g, 16.5 mm) was dissolved in 200 ml of $CH_2Cl_2$ and 75 ml of DME. To this solution was added 25 ml of dimethoxypropane followed by 450 mgs of p-toluene sulfonic acid. The solution was stirred at room temperature for 18 hours and was then washed 2x (75 ml) sat. $NaHCO_3$, 1 x (75 ml) brine, dried with $Na_2SO_4$ and concentrated to give 5.5 g of crude product. The crude product was purified by chromatography on silica gel (98 $CH_2Cl_2$:2 MeOH) to give 4.9 g of slightly impure product.

Step 3: N-(2(R)-hydroxy-1(S)-indan-(N,O-isopropylidene)yl)-3-(4-t-butyldimethylsiloxyphenyl) propaneamide)

The crude product (4.9 g, 14.54 mm) from Step 2 was dissolved in 50 ml of DMF and imidazole (9.90 g, 145.4 mm) followed by tert-butyldimethylsilyl chloride (10.96 g, 72.7 mm) was added. The mixture was stirred for 18 hours at room temperature. The DMF was then removed and the residue was partitioned between 350 ml EtOAc/100 ml $H_2O$. The organic layer was washed 1 x 100 ml sat $NaHCO_3$, 1 x 100 ml brine, dried with $Na_2SO_4$ and cmc to give 5.7 g of crude product. The crude product was purified by chromatography on silica gel. (6 Hex:1 Et0Ac to 4 Hex:1 Et0Ac) to give 4.9 g of a pure clear oil.

Step 4:

Following the same procedure as Steps 3-5 in Example 1 but substituting the product from Step 3 above, the title compound was obtained.

EXAMPLE 3

The preparation of N,N-bis-(2(R)-hydroxy-1(S)-indanyl)-2,6(R,R)-diphenylmethyl-4-amino-1,7-heptanediamide

Step 1: The preparation of N,N-bis-(2(R)-hydroxy-1(S)-indan-(N,O-isopropylidene)-yl)-2,6(R,R)-diphenylmethyl-4-(phenylmethyl)amino-1,7-heptanediamide

The ketone from Example 1, Step 5 is dissolved in 10 ml of methanol and 2 equivalents of benzyl amine is added followed by 1 eq. of sodium cyanoborohydride and some molecular sieves. The reaction is stirred at room temperature until the product is consumed. The reaction is diluted with 2 ml of 1N HCl solution and after stirring for 30 minutes is concentrated. The residue is diluted with water, washed with ethyl acetate and the organic layer is dried, filtered and concentrated to afford the crude product.

Step 2: The preparation of N,N-bis-(2(R)-hydroxy-1(S)-indanyl)-2,6(R,R)-diphenylmethyl-4-(phenylmethyl)amino-1,7-heptanediamide

The product from Step 1 above is dissolved in 5 ml of methanol and 1.2 equivalents of p-toluenesulfonic acid is added. After 18 hours the reaction is diluted with 50 ml of methylene chloride and washed with IN sodium hydroxide solution. The organic layer is dried, filtered, concentrated and the residue chromatographed to afford the desired product.

Step 3: The preparation of N,N-bis-(2(R)-hydroxy-1(S)-indanyl-2,6(R,R)-diphenylmethyl-4-amino-1.7-heptanediamide

The product from Step 2 above is dissolved in 10 ml of ethanol and an equivalent of 20% $PdOH_2$ catylyst is added. The flask is flushed with hydrogen and the reaction is stirred until the disappearance of starting material. The reaction is filtered to remove the catylyst and the filtrate is evaporated. The residue is diluted with water and extracted with methylene chloride, which is dried, filtered and concentrated. The product is obtained

by chromatography.

EXAMPLE 4

The preparation of N,N-bis-(3(R)-hydroxy-4(S)-benzothiopyranyl)-2,6(R,R)-diphenylmethyl-4-hydroxy-1,7-heptanediamide

Step 2: Preparation of N-(3(R)-hydroxy-4(S)-benzothiopyran-(N,O-isopropylidene)yl)-3-phenylpropaneamide

The title compound is prepared in the same manner as N-(2(R)-hydroxy-1(S)-indanyl-(N,O-isopropylidene)yl)(3(4-hydroxyphenyl)methyl)propaneamide in Step 2 of Example 2.

Step 3: The preparation of N,N-bis-(3(R)-hydroxy-4(S)benzothiopyran-(N,O-isopropylidene)yl)-2,-6(R,R)-diphenylmethyl-4-hydroxy-1,7-heptanediamide

To a solution of N-(3(R)-hydroxy-4(S)-benzothiopyran-N,O-isopropylidene-yl)-3-phenylpropanediamide in 5 ml of THF cooled to -78°C is added 1.1 equivalents of 1.6 N n-BuLi solution in hexanes. After 30 minutes, the reaction is added via syringe to a solution of 1:1 mixture of epibromohydrin and boron trifluoride etherate (0.5 eq). The reaction is stirred at -78°C and slowly warmed up to 0°C until it is completed. The reaction is quenched into 10% citric acid solution, washed with ethyl acetate which is dried, filtered and concentrated to afford the product after chromatography.

Step 4: The preparation of N,N-bis-(3(R)-hydroxy-4(S)-benzothiopyranyl)-2,6(R,R)-diphenylmethyl-4-hydroxy-1,7-heptanediamide

The compound from Step 1 above is treated in the same manner as described in Example 1, Step 6, to afford the title product.

EXAMPLE 5

The preparation of N,N′-bis-(3(R)-hydroxy-4-(S)-benzopyranyl)-2,6(R,R)-diphenylmethyl-4-hydroxy-1,7-heptanediamide

Step 1: The preparation of 3-((4(S)-(phenylmethyl)-2-oxazolidin)-2(R)-phenylmethyl)-4-((3-(4-(S)-(phenylmethyl)-2(R)-phenylmethyl-1-oxopropyl)-1-oxopentyl-2-oxazolidinone

To a solution of 3-(3′-phenyl-1′-oxopropyl)-4(S)-(phenylmethyl)-2-oxazolidinone in THF cooled to -78°C is added a 1 M solution of sodium hexamethyldisilazane in hexane (1.1 equivalent). The solution is warmed to -40°C and after 30 minutes, 0.5 equivalent of 3-iodo-2-iodomethylpropene is added. The temperature is maintained between -40°C and 0°C until the complete. The reaction is quenched into 10% citric acid, washed with ethyl acetate which is dried, filtered and concentrated to afford the product.

Step 2: The preparation of 2(R)-phenylmethyl-4-((3-(2(R)-phenylmethyl-propanoic acid)-pent-5-eneoic acid

To a solution of the product of Step 1, Example 5 in 30 ml of THF and 12 ml of water cooled to 0°C is added 7 eqivalents of 12 M hydrogen peroxide solution followed by the addition of 2 eq. of 1M lithium hydroxide solution. After 3 hours, 8 eq. of sodium sulfite in water (10%) is added, stirred for 30 minutes and the THF is removed by concentration. The reaction is made basic, washed with methylene chloride and the aqueous solution made acidic. The product is obtained after extracting the aqueous layer with methylene chloride followed by a standard workup.

Step 3: The preparation of N-(2(R)-hydroxy-1(S)-benzopyranyl)-2(R)-phenylmethyl-4-((3-(N′-(4(R)-tiydroxy-3(S)-benzopyranyl)-2(R)-phenylmethyl-propaneacetamid)yl)-pent-5-eneamide

To a solution of the above diacid in DMF is added 2.2 eq. of HOBT, 2.2 eq. of EDC and 2.2 eq of (2(R)-hydroxy-1(S)-aminobenzopyran. The pH of the solution is adjusted to 8-9 with triethylamine and stirred at room temperature. After the reaction is complete, the DMF is concentrated off, the residue is dissolved in 10% citric acid solution and this is washed with ethyl acetate. The organic layer is dried, filtered and concentrated to afford

the product after chromatography.

Step 4: The preparation of N,N-bis-(4(R)-hydroxy-3(S)benzopyranyl)-2,6(R,R)-diphenylmethyl-4-hydroxy-1,7-heptanediamide

The compound from Step 3 above is dissolved in methanol and cooled to -78°C. Ozone is bubbled through the solution until the characteristic blue color persists. The reaction is purged with nitrogen and excess sodium borohydride is added. The reaction is warmed to room temperature, quenched by the addition of water and the methanol is concentrated off on the rotoevaporator. The aqueous solution is extracted with chloroform and the organic layer is dried, filtered and concentrated to afford the product.

EXAMPLE 6A

The preparation of N,N′-bis-(N-2-aminomethylbenzimidazole)isoleucyl)-2,6(R,R)-diphenylmethyl-4-hydroxy-1.7-heptanediamide

In a similar manner as above but substituting N-(aminomethylbenzimidazole)-isoleucineamide for 3(R)hydroxy-4(S)-amino-benzopyran in Step 3 of Example 5, the title compound is obtained.

EXAMPLE 6B

Preparation of N,N′-bis-(4(S)-3,4-dehydro-1H-2(R)-oxobenzothiopyranyl)-2,6(R,R)-di(phenylmethyl)4-hydroxyl1,7-heptanediamide

In a similar manner as above but substituting 4-amino-3,4-dihydro-1H-benzothiopyran for 3(R)-hydroxy-4(S)-aminobenzopyran in Step 3 of Example 5, the title compound is obtained.

EXAMPLE 7

The preparation of N-(3(R)-hydroxy-4(S)-benzopyranyl)-N′-(2(R)-hydroxy-1(S)-indanyl)-2,6-(R,S)-diphenylmethyl-4(R,S)-hydroxy-1,7-heptanamide

Step 1: Preparation of N-(2(R)-hydroxy-1(S)-indan-(N,O-isopropylidene)yl)-2(R)-phenylmethyl-4-Iodomethyl-pent-5-eneamide

To a solution of N-(2(R)-hydroxy-1(S)-indan(N,O-isopropylidene)yl)-3-phenyl-propaneamide in 5 ml of THF cooled to 78°C is added 1.1 eq of a 1.6 N n-BuLi solution in hexanes. After 30 minutes, 3 eq. of 3-iodo-2-iodomethyl-1-propene is added. The solution is stirred at -78°C for 1 hour, warmed to 0°C, stirred another hour and then quenched by pouring into 30 ml of 10% citric acid solution. This is washed with ethyl acetate (2 x 40 ml) and the organic layer is washed with satd. brine soluton, dried, filtered and concentrated. The crude residue is chromatographed to afford the product.

Step 2: N-(2(R)-hydroxy-1(S)-indanyl)-2(R)-phenylmethyl-4-((3-(N′-(2(R)-hydroxy-1(S)-benzopyranyl)-2(R)-phenylmethylpropaneacetamid)yl)-pent-5-eneamide

To a solution of N(3(R)-hydroxy-4(S)-benzopyranyl-(N,O-isopropylidene)yl)-3-phenylpropaneamide in 5 ml of THF cooled to -78°C is added 1.1 eq. of a 1.6 N n-BuLi solution in hexanes. After 30 minutes, 1 eq. of N-(2(R)-hydroxy-1(S)-indan-(N,O-isopropylidene)-yl)-2(R)-phenylmethyl-4-Iodomethyl-pent-5-eneamide in 5 ml of THF is added. The solution is stirred at -78°C for 1 hour, warmed to 0°C, stirred another hour and then quenched by pouring into 30 ml of 10% citric acid solution. This is washed with ethyl acetate and the organic layer is washed with satd. brine solution, dried filtered and concentrated. The crude residue is chromatographed to afford the product.

Step 3: The preparation of N-(3(R)-hydroxy-4(S)-benzopyranyl)-N′-(2(R)-hydroxy-1(S)-indanyl)-2,6(R,R)-diphenylmethyl-4(RS)-hydroxy-1,7-heptanamide

The compound from Step 2 above is converted to the title compound following Steps 4, 5, and 6 of Example 1.

EXAMPLE 8

The preparation of N-(2(R)-hydroxy-1(S)-indanyl)-N′-(3(R)-hydroxy-4(S)-benzopyranyl)-2(R)-phenylmethyl-6(R)-(4-hydroxyphenyl)methyl-4(RS)-hydroxy-1,7-heptanamide

Step 1: N-(2(R)-hydroxy-1(S)-indanyl)-2(R)-phenylmethyl-4((3-(N′-(3(R)-hydroxy-4(S)-benzopyranyl)-2(R)-(4-hydroxyphenyl)methylpropaneacetamid)yl)-pent-5-eneamide

To a solution of N-(3(R)-hydroxy-4(S)-benzopyranyl)-(N,O-isopropylidene)yl)-3(4-(t-butyldimethylsilyl)oxyphenyl)-propaneamide in 5 ml of THF cooled to -78°C is added 1.1 eq. of a 1.6 N n-BuLi solution in hexanes. After 30 minutes, 1 eq. of N-(2(R)-hydroxy-1(S)-indan-(N,O-isopropylidene)yl)-2(R)-phenylmethyl-4-Iodomethyl-pent-5-eneamide in 5 ml of THF is added. The solution is stirred at -78°C for 1 hour, warmed to 0°C, stirred another hour and then quenched by pouring into 30 ml of 10% citric acid solution. This is washed with ethyl acetate and the organic layer is washed with saturated brine solution, dried, filtered and concentrated. The crude residue is chromatographed to afford the product.

Step 2: The preparation of N-(2(R)-hydroxy-1(S)-indanyl),N′-(3(R)-hydroxy-4(S)-benzopyranyl)-2(R)-phenylmethyl-6(R)-(4-hydroxyphenyl)methyl-4(RS)-hydroxy-1,7-heptanamide

The compound from Step 1 above is converted to the title compound following Steps 4, 5 and 6 of Example 1.

EXAMPLE 9

Preparation of N,N′-bis-(2(R)-hydroxy-1(S)-indanyl)-2,6(R,R)-di((4-(2-hydroxyethyloxy)phenylmethyl-4-hydroxy-1,7-heptanediamide

Step 1: Preparation of N,N′-bis-(2(R)-hydroxy-1-(S)-indanyl)-2,6(R,R)-di((4-(2-allyloxy)phenyl)methyl)-4-hydroxy-1,7-heptanediamide

To a solution of N,N′-bis-(2(R)-hydroxy-1-(S)-indanyl)-2,6(R,R)-di((4-hydroxy)phenyl)methyl)-4-hydroxy-1,7-heptandiamide in dioxane is added 10 eq. of allyl bromide and 5 eq. of cesium carbonate. The reaction is heated to 90°C. When the reaction is the complete, the precipitate is filtered off, the dioxane is concentrated to dryness and the residue is diluted with water which is washed with ethyl acetate. The organic phase is dried, filtered and concentrated to afford the product.

Step 2: Preparation of N,N′-bis-(2(R)-hydroxy-1-(S)-indanyl)-2,6(R,R)-di((4-((2-hydroxy)ethoxy)phenyl)methyl)-4-hydroxy-1,7-heptanediamide

The product from Step 1 above is dissolved in methanol, cooled to -78°C and excess ozone is bubbled through the reaction until a blue color persists. The flask is purged with nitrogen to remove any ozone and excess sodium borohydride is added. The reaction is warmed to room temperature and then water is added. The methanol is concentrated off on the rotoevaporater and the aqueous residue washed with ethyl acetate. The organic layer is dried, filtered and concentrated to afford the product.

EXAMPLE 10

The preparation of N,N′-bis-(2(R)-hydroxy-1(S)-indanyl)-2,6(R,R)-di((4-(2-(4-morpholinyl)-ethoxy)phenyl)methyl-4-hydroxy-1,7-heptanediamide

Step 1: Preparation of N,N′-bis-(2(R)-hydroxy-1-(S)-indanyl)-2,6(R,R)-di((4-(2-(4-morpholinyl)ethoxy)phenyl)methyl)-4-hydroxy-1,7-heptanediamide

To a solution of N,N′-bis-(2(R)-hydroxy-1-(S)-indanyl)-2,6(R,R)-di((4-hydroxyphenyl)methyl)4-hydroxy-1,7-heptanediamide in dioxane is added 20 eq. of chloroethyl morpholine and 10 eq. of cesium carbonate. The reaction is heated to 90°C. When the reaction is the complete, the precipitate is filtered off, the dioxane is concentrated to dryness and the residue is diluted with water which is washed with ethyl acetate. The organic phase is dried, filtered and concentrated to afford the product.

EXAMPLE 11

Assay for Inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30°C for 1 hour. Various concentrations of inhibitor in 1.0 ul DMAO were added to 25 ul of the peptide solution in water. The reaction is initiated by the addition of 15 ul of 0.33 nM protease (0.11 ng) in a solution of 0.133 M Na acetate pH 5.5 and 0.26% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. The product of Example 1 showed $IC_{50}$ value of about 0.7nM.

While the foregoing specification teaches the principles of the present invention, with examples provided for the purpose of illustration, it will be understood that the practice of the invention encompasses all of the usual variations, adaptations, or modifications, as come within the scope of the following claims and its equivalents.

## Claims

1. A compound of the formula

J-B-B-G-B-B-J     I

wherein
G is

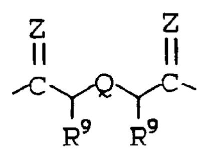

wherein Z is O or S, and
Q is

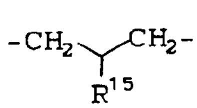

or

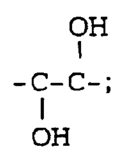

$R^9$ is independently
1) hydrogen;
2)

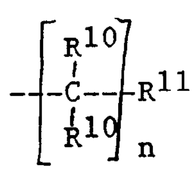

;

3) $-OR^{11}$,
4) $-N(R^{11})_2$,
5) $C_{1-4}$ alkylene-$R^{11}$ or
6) $-s(R^{11})$;
wherein n is 0-5 and $R^{10}$ is independently
a) hydrogen,

b) hydroxy, or
c) $C_{1-4}$-alkyl; $R^{11}$ is
a) hydrogen,
b) $C_6$-$C_{10}$ aryl, unsubstituted or substituted with one or more of
i) halo,
ii) hydroxy,
iii) $-NH_2$, $-NO_2$, -NHR, or $-NR_2$, wherein R is H, or $C_{1-4}$ alkyl,
iv) $C_{1-4}$ alkyl,
v) $C_{1-3}$ alkoxy,
vi) -COOR,
vii)

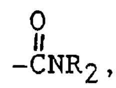

viii) $-CH_2NR_2$,
ix)

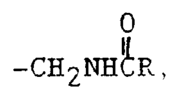

x) CN,
xi) $CF_3$,
xii)

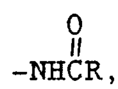

xiii) aryl $C_{1-3}$ alkoxy,
iv) aryl,
xv) $-NRSO_2R$,
xvi) $-OP(O)(OR_x)_2$ wherein $R_x$ is H or aryl,
xvii)

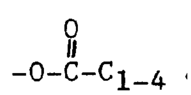

alkyl substituted with one or more of amine or quaternary amine, or
xviii) $-R^{12}$, as defined below;
c) 5 or 6 membered heterocycle including up to 3 heteroatoms selected from N, 0, and S, any of which heterocycle may be unsubstituted or substituted with one or more of
i) halo,
ii) hydroxy,
iii) $-NH_2$, -NHR, $-NR_2$,
iv) $C_{1-4}$ alkyl,
v) $C_{1-3}$ alkoxy,
vi) -COOR,
vii)

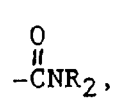

viii) $-CH_2NR_2$,
ix)

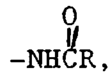

x) -CN,
xi) $CF_3$,
xii) $-NHSO_2R$,
xiii) $-OP(O)(OR_x)_2$ wherein $R_x$ is H or aryl,
xiv)

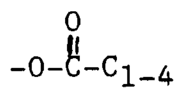

alkyl substituted with one or more of amine or quaternary amine, or
xv) $-R^{12}$;

d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)

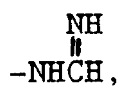

v)

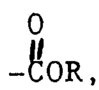

vi) -SR, or arylthio,
vii) $-SO_2NHR$,
vii) $C_{1-4}$ alkyl sulfonyl amino or aryl sulfonyl amino,
viii) -CONHR,
ix)

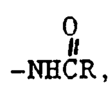

x) -OR,
xi) aryl $C_{1-3}$ alkoxy,
xii) aryl, or
xiii) aryl substituted with $R^{12}$;

e) $C_{3-7}$ cycloalkyl unsubstituted or substituted with one or more of
i) hydroxy,
ii) $C_{1-4}$alkyl,
iii) $-NH_2$, -NHR, $-NR_2$,
iv)

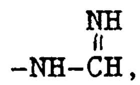

v)

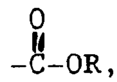

vi) -SR,
vii) $-SO_2NH_2$,
viii) alkyl sulfonylamino or aryl sulfonylamino,
ix) -CONHR,
x)

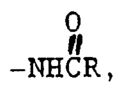

or
xi) $-R^{12}$;

f) a 5- to 7-membered carbocyclic or 7- to 10-membered bicyclic carbocyclic ring which is either saturated or unsaturated, the carbocyclic ring being unsubstituted or substituted with one or more of
i) halo
ii) -OR, wherein R is H or $C_{1-4}$ alkyl,
iii)

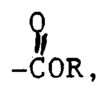

iv)

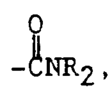

v) $-CH_2NR_2$,
vi) $-SO_2NR_2$ or $-S(O)_y$ R wherein y is 0,1 or 2,
vii) $-NR_2$,
viii)

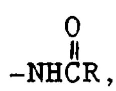

ix) $C_{1-4}$alkyl,
x) phenyl,
xi) $-CF_3$,
xii)

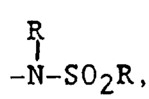

or

xiii) $-R^{12}$; $R^{12}$ is

a) $-X-(CH_2)_m--XR^{13}$ where X is independently -O-,-S-, or NR; m is 2-5, and $R^{13}$ is independently hydrogen or

i) $C_{1-6}$ alkyl,

ii) $C_{1-6}$ alkyl substituted with one or more of

(a) $C_{1-3}$ alkoxy,

(b) -OH,

(c) $-NR_2$ where R is hydrogen or $C_{1-4}$ alkyl;

iii) aromatic heterocycle unsubstituted or substituted with one or more of

(a) $C_{1-4}$ alkyl, or

(b) $-NR_2$;

b) $-X-(CH_2)_m-NR^{13}R^{13}$ wherein $R^{13}$ is the same or different and joined together to form a 5-7 member heterocycle containing up to two additional heteroatoms selected from

(a) -NR,

(b) -O-.

(c) -S-,

(d)

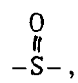

(e) $-SO_2-$;

c) $-(CH_2)_q--NR^{13}R^{13}$ wherein q is 1-5, and $R^{13}$ is defined above;

$R^{15}$ is -OH or $-NHR^{16}$, wherein $R^{16}$ is -H,

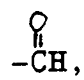

$-C_{1-4}-$ alkyl, or -COOR; and

B is, independently, absent, or

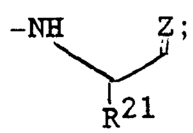

where $R^{21}$ is

a) $-CH(CH_3)_2$,

b) $-CH(CH_3)(CH_2CH_3)$,

c) -Phenyl,

d) $-CH_2CONH_2$, or

e) $-CH_2CH_2OH$;

J is

1) $YR^{17}$ wherein:

Y is O or NH, and

$R^{17}$ is

a), H;

b) $C_{1-6}$ alkyl, unsubstituted or substituted with one or more of

i) $-NR_2$,

ii) -OR,

iii) $-NHSO_2C_{1-4}$ alkyl,

iv) $-NHSO_2$ aryl or $-NHO_2$(dialkylaminoaryl),
v) $-CH_2OR$,
vi) $-C_{1-4}$ alkyl,
vii)

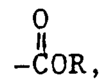

viii)

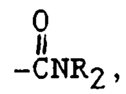

ix) 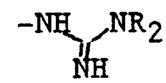 or 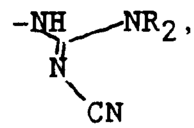

x) 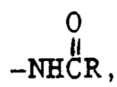

xi)

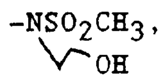

xii)

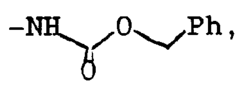

xiii) $-NR_3\oplus A\ominus$ wherein $A\ominus$ is a counterion,
xiv) $-NR^{18}R^{19}$ wherein $R^{18}$ and $R^{19}$ are the same or different and are $C_{1-5}$ alkyl joined together directly to form a 5-7 membered heterocycle containing up to one additional heteroatom selected from -O-, -S-, or -NR-,
xv) aryl,
xvi) -CHO,
xvii) $-OP(O)(OR_x)_2$ wherein $R_x$ is H or aryl, or
xvii)

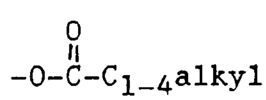

substituted with one or more of amine or quaternary amine, or $-O-[(CH_2)_mO]_n-R$, or $-OP(O)(OR_x)_2$;
2) $N(R^{17})_2$;
3) $-NR^{18}R^{19}$ wherein $R^{18}$ and $R^{19}$ are defined above; or
4)

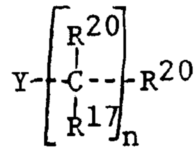

wherein:
Y, $R^{17}$ and n are defined above, and

$R^{20}$ is
a) hydrogen;
b) aryl unsubstituted or substituted with one or more of
i) halo,
ii) -OR, wherein R is H or $C_{1-4}$ alkyl,
iii)

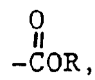

iv)

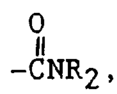

v) $-CH_2NR_2$,
vi) $-SO_2NR_2$,
vii) $-NR_2$,
viii)

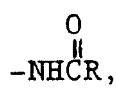

xi) $C_{1-4}$ alkyl,
x) phenyl,
xi) $-CF_3$,
xii)

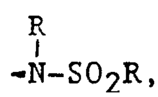

xiii) $-C_{1-4}$ alkyl $-NR_2$,
xiv) $-OP(O)(OR_x)_2$ wherein $R_x$ is H or aryl, or
xv)

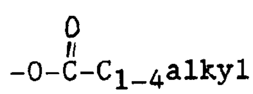

substituted with one or more of amine or quaternary amine or $-OP(O)(OR_x)_2$;
c) A 5-7 membered heterocyclic ring or, 7-10 membered bicyclic heterocyclic ring which is saturated or unsaturated, the ring being unsubstituted or ,substituted with one or more of
i) halo,
ii) -OR, wherein R is H, $C_{1-4}$alkyl, or $C_{1-4}$alkenyl,

iii)

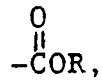

iv)

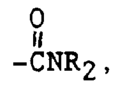

v) $-CH_2NR_2$,
vi) $-SO_2NR_2$,
vii) $-NR_2$,
viii)

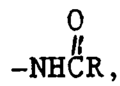

xi) $C_{1-4}$ alkyl,
x) phenyl
xi) $-CF_3$,
xii)

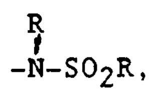

xiii) phenyl $C_{1-4}$ alkyl,
xiv) $-OP(O)(OR_x)_2$ wherein $R_x$ is H or aryl,
xiv)

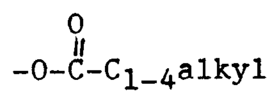

substituted with one or more of amine or quaternary amine, or $-OP(O)(OR_x)_2$, or $-O[(CH_2)_mO]_n-R$, or
xvi)

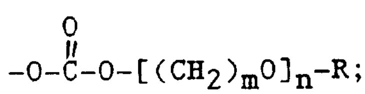

d) A 5 to 7 membered carbocyclic or 7-10 membered bicyclic carbocyclic ring which is either saturated or unsaturated, the carbocyclic ring being unsubstituted or substituted with one or more of
i) halo,
ii) -OR, wherein R is H or $C_{1-4}$ alkyl,
iii)

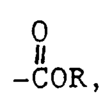

iv) $-CNR_2$,

v)

$$-\overset{O}{\overset{\|}{C}}H_2NR_2,$$

vi) $-SO_2NR_2$,
vii) $-NR_2$,
viii)

$$-NH\overset{O}{\overset{\|}{C}}R,$$

xi) $C_{1-4}$ alkyl,
x) phenyl,
xi) $-CF_3$,
xii)

$$-\overset{R}{\overset{|}{N}}-SO_2R,$$

xiii) $-OP(O)(OR_x)_2$ wherein $R_x$ is H or aryl,
xiv)

$$-O-\overset{O}{\overset{\|}{C}}-C_{1-4}alkyl$$

substituted with one or more of amine or quaternary amine, $-OP(O)(OR_x)_2$, or $-O-[(CH_2)_mO]n-R$, or xv)

$$-O-\overset{O}{\overset{\|}{C}}-O-[(CH_2)_mO]_n-R;$$

or pharmaceutically acceptable salts thereof

2. The compound of Claim 1, wherein
G is

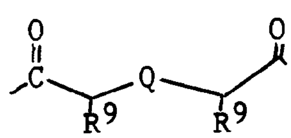

3. The compound of Claim 2, wherein
B is absent or present once and Q is

$$CH_2-\underset{OH}{\underset{|}{C}}H-CH_2- \text{ or } CH_2-\underset{N-H_2}{\underset{|}{C}H}-CH_2$$

4. The compound of Claim 3, wherein B is absent and $R^{20}$ is a substituted 5 to 7 membered carbocyclic or heterocyclic or 7 to 10 membered bicyclic carbocyclic or heterocyclic ring which is either saturated or unsaturated.

5. The compound of Claim 4, wherein $R^{20}$ is indan substituted with one or more of hydroxy or amine, or benzothiopyranyl substituted one or more times with hydroxy.

6. A compound which is
N,N′-bis-(2(R)-hydroxy-1(S)-indanyl)-2,6(R,R)-di(phenylmethyl)-4-hydroxy-1,7-heptanediamide,
N,N′-bis-(3(S)-hydroxy-4(S)-benzopyranyl)-2,6(R,R)di(phenylmethyl)-4-hydroxy-1,7-heptanediamide,
N,N′-bis-(3(S)-hydroxy-4(S)-benzothiopyranyl)-2,6(R,R)-di(phenylmethyl)-4-hydroxy-1,7-heptanediami de,
N,N′-bis-(4(S)-3,4-dihydro-1H-2-benzothiopyranyl)-2,6(R,R)-di(phenylmethyl)-4-hydroxy-1,7-heptanedi amide,
N,N′-bis-(4(S)-3,4-dihydro-1H-2(R)-oxobenzothiopyranyl)-2,6(R,R)-di(phenylmethyl)-4-hydroxy-1,7-hep tanediamide,
N,N′-bis-(4(S)-3,4-dihydro-1H-2,2-dioxobenzothiopyranyl)-2,6(R,R)-di(phenylmethyl)-4-hydroxy-1,7-he ptanediamide,
N,N′-bis-(2(S),3(R)-dihydroxy-1(S)-indanyl)-2,6(R,R)-di(phenylmethyl)-4-hydroxy-1,7-heptanediamide,
N,N′-bis-(3(R)-hydroxy-1(S)-indanyl)-2,6(R,R)-di(phenylmethyl)-4-hydroxy-1,7-heptanediamide,
N,N′-bis-(3(R)-amino-2(R)-hydroxy-1(S)-indanyl)-2,6(R,R)-di(phenylmethyl)-4-hydroxy-1,7-heptanedia mide,
N,N′-bis-(4(R)-hydroxy-2(S)-methyl-1(R)-cyclopentyl)-2,6(R,R)-di(phenylmethyl)-4-hydroxy-1,7-heptan ediamide,
N,N′-bis-(3(S)-hydroxy-4(S)-benzothiopyranyl-1(S)-oxido)-2,6(R,R)-di(phenylmethyl)-4-hydroxy-1,7-hep tanediamide,
N,N′-bis-(2(R)-hydroxy-5(R)-methyl-1(S)-cyclopentyl)-2,6(R,R)-di(phenylmethyl)-4-hydroxy-1,7-heptan ediamide,
N,N′-bis-(4(R)-benzothiopyranyl-1(S)-oxido)-2,6(R,R)di(phenylmethyl)-4-hydroxy-1,7-heptanediamide,
N,N′-bis-(3-methyl-2(S)-amino-1-hydroxybutyl)-2,6-(R,R)-di(phenylmethyl)-4-hydroxy-1,7-heptanediam ide,
N,N′-bis-(2(S)-amino-1-hydroxyphenethyl)-2,6(R,R)-di(phenylmethyl)-4-hydroxy-1,7-heptanediamide,
N,N′-bis-(2(R)-hydroxy-1(S)-indanyl)-2,6(R,R)-((4-(2-(4-morpholinyl)ethoxy)phenyl)methyl)-4-hydroxy-1 ,7-heptanediamide,
N,N′-bis-(3(S)-hydroxy-4(S)-benzopyranyl)-2,6(R,R)-((4-(2-(4-morpholinyl)ethoxy)phenyl)methyl)-4-hyd roxy-1,7-heptanediamide,
N,N′-bis-(3(S)-hydroxy-4(S)-benzothiopyranyl)-2,6-(R,R)-((4-(2-(4-morpholinyl)ethoxy)phenyl)methyl)-4 -hydroxy-1,7-heptanediamide,
N,N′-bis-(4(S)-3,4-dihydro-1H-2-benzothiopyranyl)-2,6-(R,R)-((4-(2-(4-morpholinyl)ethoxy)phenyl)meth yl)-4-hydroxy-1,7-heptanediamide,
N,N′-bis-(4(S)-3,4-dihydro-1H-2(R)-oxobenzothiopyranyl)-2,6(R,R)-((4-(2-(4-morpholinyl)ethoxy)phenyl) methyl)-4-hydroxy-1,7-heptanediamide,
N,N′-bis-(4(S)-3,4-dihydro-1H-2,2-dioxobenzothiopyranyl)-2,6(R,R)-((4-(2-(4-morpholinyl)ethoxy)phenyl )methyl)-4-hydroxy-1,7-heptanediamide,
N,N′-bis-(2(S),3(R)-dihydroxy-1(S)-indanyl-2,6(R,R)-((4-(2-(4-morpholinyl)ethoxy)phenyl)methyl)-4-hyd roxy-1,7-heptanediamide,
N,N′-bis-(3(R)-hydroxy-1(S)-indanyl-2,6(R,R)-((4-(2-(4-morpholinyl)ethoxy)phenyl)methyl)-4-hydroxy-1 ,7-heptanediamide,
N,N′-bis-(3(R)-amino-2(R)-hydroxy-1(S)-indanyl)-2,6(R,R)-((4-(2-(4-morpholinyl)ethoxy)phenyl)methyl) -4-hydroxy-1,7-heptanediamide,
N,N′-bis-(4(R)-hydroxy-2(S)-methyl-1(R)-cyclopenyl)-2,6(R,R)-((4-(2-(4-morpholinyl)ethoxy)phenyl)met hyl)-4-hydroxy-1,7-heptanediamide,
N,N′-bis-(3(R)-hydroxy-4(S)-benzothiopyranyl-1(S)-oxido)-2,6(R,R)-((4-(2-(4-morpholinyl)ethoxy)phenyl )methyl)-4-hydroxy-1,7-heptanediamide,
N,N′-bis-(2(R)-hydroxy-5(R)-methyl-1(S)-cyclopentyl)-2,6(R,R)-((4-(2-(4-morpholinyl)ethoxy)phenyl)me thyl)-4-hydroxy-1,7-heptanediamide,
N,N′-bis-(4(R)-benzothiopyranyl-1(S)-oxido)-2,6(R,R)-((4-(2-(4-morpholinyl)ethoxy)phenyl)methyl)-4-h ydroxy-1,7-heptanediamide,
N,N′-bis-(2(R)-hydroxy-1(S)-indanyl)-2,6(R,R)-di((4-((2-hydroxy)ethoxy)phenyl)methyl)-4-hydroxy-1,7-heptanediamide,
N,N′-bis-(3(S)-hydroxy-4(S)-benzopyranyl)-2,6(R,R)-di ((4-((2-hydroxy)ethoxy)phenyl)methyl)-4-hydroxy-1,7-heptanediamide,

N,N′-bis-(3(S)-hydroxy-4(S)-benzothiopyranyl)-2,6-(R,R)-di((4-((2-hydroxy)ethoxy)phenyl)methyl)-4-hydroxy-1,7-heptanediamide,
N,N′-bis-(4(S)-3,4-dihydro-1H-2-benzothiopyranyl)-2,6 (R,R)-di((4-((2-hydroxy)ethoxy)phenyl)methyl)-4-hydroxy-1,7-heptanediamide,
N,N′-bis-(4(S)-3,4-dihydro-1H-2(R)-oxobenzothiopyranyl)-2,6(R,R)-di((4-((2-hydroxy)ethoxy)phenyl)methyl)-4-hydroxy-1,7-heptanediamide,
N,N′-bis-(4(S)-3,4-dihydro-1H-2,2-dioxobenzothiopyranyl)-2,6(R,R)-di((4-((2-hydroxy)ethoxy)phenyl)methyl)-4-hydroxy-1,7-heptanediamide,
N,N′-bis-(2(S),3(R)-dihydroxy-1(S)-indanyl)-2,6(R,R)-di((4-((2-hydroxy)ethoxy)phenyl)methyl)-4-hydroxy-1,7-heptanediamide,
N,N′-bis-(3(R)-hydroxy-1(S)-indanyl)-2,6(R,R)-di((4-((2-hydroxy)ethoxy)phenyl)methyl)-4-hydroxy-1,7-heptanediamide,
N,N′-bis-(3(R)-amino-2(R)-hydroxy-1(S)-indanyl)-2,6(R,R)-di((4-((2-hydroxy)ethoxy)phenyl)methyl)-4-hydroxy-1,7-heptanediamide,
N,N′-bis-(4(R)-hydroxy-2(S)-methyl-1(R)-cyclopentyl)-2,6(R,R)-di((4-((2-hydroxy)ethoxy)phenyl)methyl)-4-hydroxy-1,7-heptanediamide,
N,N′-bis-(3(S)-hydroxy-4(S)-benzothiopyranyl-1(S)-oxido)-2,6(R,R)-di((4-((2-hydroxy)ethoxy)phenyl)methyl)-4-hydroxy-1,7-heptanediamide,
N,N′-bis-(2(R)-hydroxy-5(R)-methyl-1(S)-cyclopentyl)-2,6(R,R)-di((4-((2-hydroxy)ethoxy)phenyl)methyl)-4-hydroxy-1,7-heptanediamide,
N,N′-bis-(4(R)-benzothiopyranyl-1(S)-oxido-2,6(R,R)-di((4-((2-hydroxy)ethoxy)phenyl)methyl)-4-hydroxy-1,7-heptanediamide,
N,N′-bis-(3-methyl-2(S)-amino-1-hydroxybutyl)-2,6-(R,R)-di((4-((2-hydroxy)ethoxy)phenyl)methyl)-4-hydroxy-1,7-heptanediamide,
or
N,N′-bis-(2(S)-amino-1-hydroxyphenethyl)-2,6(R,R)-di((4-((2-hydroxy)ethoxy)phenyl)methyl)-4-hydroxy-1,7-heptanediamide,
or
pharmaceutically acceptable salts thereof.

7. The compound, which is
N,N′-bis-(2(R)-hydroxy-1(S)-indanyl)-2,6(R,R)-di(phenylmethyl)-4-hydroxy-1,7-heptanediamide, or pharmaceutically acceptable salt thereof.

8. The compound, which is
N,N′-bis-(4(S)-3,4-dihydro-1H-2(R)-oxobenzothiopyranyl)-2,6(R,R)-di(phenylmethyl)-4-hydroxy-1,7-heptanediamide, or pharmaceutically acceptable salt thereof.

9. The compound, which is
N,N′-bis-(2(R)-hydroxy-1(S)-Indanyl)-2,6(R,R)-di((4-2-(4-morpholinyl)ethoxy)phenyl)methyl)-4-hydroxy-1,7-heptanediamide,
or pharmaceutically acceptable salt thereof.

10. The compound, which is
N,N′-bis-(2(R)-hydroxy-1(S)-indanyl)-2,6(R,R)-di((4-((2-hydroxy)ethoxy)phenyl)methyl)-4-hydroxy-1,7-heptanediamide,
or pharmaceutically acceptable salts thereof.

11. A pharmaceutical composition comprising a compound as claimed in any of Claims 1 to 10 and a pharmaceutically acceptable carrier.

12. The use of a compound as claimed in any of Claims 1 to 10 for the manufacture of a medicament for inhibiting HIV protease or for the prevention or treatment of infection by HIV, or the treatment of AIDS.